# EUROPEAN PATENT SPECIFICATION

(11) **EP 2 539 439 B1**
(45) Date of publication and mention of the grant of the patent: **17.10.2018**
(21) Application number: 11747958.4
(22) Date of filing: 23.02.2011
(51) Int. Cl.: A61K 35/545, G01N 33/50, C12N 5/074, A61K 38/19, A61K 38/20, A61P 9/00

(54) **MODULATION OF ANGIOGENESIS**
ANGIOGENESE-MODULATION
MODULATION DE L'ANGIOGENÈSE

(30) Priority: 25.02.2010 US 308103 P
(43) Date of publication of application: 02.01.2013
(73) Proprietor: ABT Holding Company, Cleveland, OH 44115 (US)
(72) Inventor: WODA, Juliana, Megan, Shaker Heights, OH 44120 (US); TING, Anthony, E., Shaker Heights, OH 44122 (US); LEHMAN, Nicholas, A., Solon, OH 44139 (US)
(74) Representative: Cornish, Kristina Victoria Joy
(86) International application number: PCT/US2011/025846
(87) International publication number: WO 2011/106365

(56) References cited:
- WO-A1-2008/148105
- WO-A2-2004/039248
- US-A- 4 303 796
- US-A1- 2005 181 502
- US-A1- 2006 003 323
- US-A1- 2006 036 086
- US-A1- 2007 184 012
- US-A1- 2007 253 937
- US-A1- 2007 253 937
- US-A1- 2008 274 088
- US-A1- 2008 274 088
- DATABASE WPI Week 200523 Thomson Scientific, London, GB; AN 2005-218303 XP002703633, & JP 2005 065661 A (SAKURAI D) 17 March 2005 (2005-03-17)
- DATABASE BIOSIS [Online] BIOSCIENCES INFORMATION SERVICE, PHILADELPHIA, PA, US; September 2005 (2005-09), SALES KURT J ET AL: "A novel angiogenic role for prostaglandin F-2 alpha-FP receptor interaction in human endometrial adenocarcinomas", XP002703668, Database accession no. PREV200510294799 & SALES KURT J ET AL: "A novel angiogenic role for prostaglandin F-2 alpha-FP receptor interaction in human endometrial adenocarcinomas", CANCER RESEARCH, vol. 65, no. 17, September 2005 (2005-09), pages 7707-7716, ISSN: 0008-5472
- LEI Y ET AL: "THERAPEUTIC ANGIOGENESIS. DEVISING NEW STRATEGIES BASED ON PAST EXPERIENCE", BASIC RESEARCH IN CARDIOLOGY, STEINKOPFF, DARMSTADT, DE, vol. 99, no. 2, 1 January 2004 (2004-01-01), pages 121-132, XP008065176, ISSN: 0300-8428, DOI: 10.1007/S00395-004-0447-X

## Description

### FIELD OF THE INVENTION

The disclosure concerns methods for treating pathological conditions that can be improved by providing angiogenesis. The disclosure is generally directed to providing angiogenesis by administering cells that express and/or secrete one or more pro-angiogenic factors. The disclosure is also directed to drug discovery methods to screen for agents that modulate the ability of the cells to express and/or secrete one or more pro-angiogenic factors. The disclosure is also directed to cell banks that can be used to provide cells for administration to a subject, the banks comprising cells having desired levels of expression and/or secretion of one or more pro-angiogenic factors. The disclosure is also directed to compositions comprising cells having specific desired levels of expression and/or secretion of one or more pro-angiogenic factors, such as pharmaceutical compositions. The disclosure is also directed to diagnostic methods conducted prior to administering the cells to a subject to be treated, including assays to assess the desired potency of the cells to be administered. The disclosure is further directed to post-treatment diagnostic assays to assess the effect of the cells on a subject being treated. The cells described herein are non-embryonic stem, non-germ cells that can be characterized by one or more of the following: extended replication in culture and express markers of extended replication, such as telomerase, markers of pluripotentiality, and broad differentiation potential, without being transformed.

### SUMMARY OF THE INVENTION

The aspects of the invention for which protection is sought are defined in the claims.

According to a first aspect, the invention provides cells having expression and/or secretion of the pro-angiogenic factors VEGF, CXCL5 and IL-8 for use in preventing or treating an ischemic condition in a subject, the cells being non-embryonic stem, non-germ cells that express one or more of oct4, telomerase, rex-1, or rox-1 and can differentiate into cell types of at least two of endodermal, ectodermal, and mesodermal germ layers, wherein, prior to administration, said cells have been assayed for having expression and/or secretion of the pro-angiogenic factors VEGF, CXCL5 and IL-8. The cells may be allogeneic. The ischemic condition may be a condition selected from the group consisting of acute myocardial infarction, chronic heart failure, peripheral vascular disease, stroke, chronic total occlusion, renal ischemia, and acute kidney injury. The subject may be human. The cells may be multipotent progenitor cells.

According to a second aspect, the invention provides a method for constructing a cell bank, said method comprising assaying cells for having expression and/or secretion of the pro-angiogenic factors VEGF, CXCL5 and IL-8, expanding and storing, for future administration to a subject, cells that have the expression and/or secretion of the pro-angiogenic factors VEGF, CXCL5 and IL-8, the cells being non-embryonic stem, non-germ cells that express one or more of oct4, telomerase, rex-1, or rox-1 and can differentiate into cell types of at least two of endodermal, ectodermal, and mesodermal germ layers. The cells may be multipotent progenitor cells.

According to a third aspect, the invention provides an *in vitro* method for drug discovery, said method comprising exposing cells that have expression and/or secretion of the pro-angiogenic factors VEGF, CXCL5 and IL-8, to an agent to assess the effect of the agent on the ability of the cells to express and secrete the pro-angiogenic factors VEGF, CXCL5 and IL-8, the cells being non-embryonic stem, non-germ cells that express one or more of oct4, telomerase, rex-1, or rox-1 and/or can differentiate into cell types of at least two of endodermal, ectodermal, and mesodermal germ layers. The cells may have been assayed for having expression and/or secretion of the pro-angiogenic factors VEGF, CXCL5 and IL-8. The cells may be multipotent progenitor cells.

According to a fourth aspect, the invention provides an *in vitro* method for increasing the expression of the pro-angiogenic factors VEGF, CXCL5 and IL-8 in a stem cell, the method comprising exposing the cell to a combination of TNF-α, IL-1β, and IPNγ, or to a prostaglandin F analog wherein said cells are non-embryonic stem, non-germ cells that express one or more of oct4, telomerase, rex-1, or rox-1 and can differentiate into cell types of at least two of endodermal, ectodermal, and mesodermal germ layers, wherein the prostaglandin F analog is latanoprost. The cells may be multipotent progenitor cells.

The disclosure is broadly directed to methods for providing angiogenesis.

The disclosure is also directed to methods for providing one or more pro-angiogenic factors to provide angiogenesis.

Pro-angiogenic factors include, but are not limited to, FGF, VEGF, VEGFR, NRP-1, Ang1, Ang2, PDGF (BB-homodimer), PDGFR, TGF-β, endoglin, TGF-β receptors, MCP-1, Integrins αᵥβ₃, αᵥβ₃, α₅β₁, VE-Cadherin, CD31, ephrin, plasminogen activators, plasminogen activator inhibitor-1, eNOS, COX-2, AC133, Id1/Id3, Angiogenin, HGF, Vegf, Il-1 alpha, 11-8, 11-6, Cxcl5, Fgfα, Fgfβ, Tgfα, Tgfβ, MMPs (including mmp9), Plasminogen activator inhibitor-1, Thrombospondin, Angiopoietin 1, Angiopoietin 2, Amphiregulin, Leptin, Endothelin-1, AAMP, AGGF1, AMOT, ANGLPTL3, ANGPTL4, BTG1, IL-1β, NOS3, TNFSF12, and VASH2.

Providing angiogenesis can be achieved by administering cells naturally (i.e., non-recombinantly) expressing and/or secreting one or more pro-angiogenic factors or medium conditioned by the cells. Cells include, but are not limited to, cells that are not embryonic stem cells and not germ cells, having some characteristics of embryonic stem cells, but being derived from non-embryonic tissue, and expressing and/or secreting one or more pro-angiogenic factors. The cells may naturally express/secrete one or more pro-angiogenic factors (i.e., not genetically or pharmaceutically modified to activate expression and/or secretion). However, natural expressors can be genetically or pharmaceutically modified to increase potency.

The cells may express pluripotency markers, such as oct4. They may also express markers associated with extended replicative capacity, such as telomerase. Other characteristics of pluripotency can include the ability to differentiate into cell types of more than one germ layer, such as two or three of ectodermal, endodermal, and mesodermal embryonic germ layers. Such cells may or may not be immortalized or transformed in culture. The cells may be highly expanded without being transformed and also maintain a normal karyotype. For example, the non-embryonic stem, non-germ cells may have undergone at least 10-40 cell doublings in culture, such as 50, 60, or more, wherein the cells are not transformed and have a normal karyotype. The cells may differentiate into at least one cell type of each of two of the endodermal, ectodermal, and mesodermal embryonic lineages and may include differentiation into all three. Further, the cells may not be tumorigenic, such as not producing teratomas. If cells are transformed or tumorigenic, and it is desirable to use them for infusion, such cells may be disabled so they cannot form tumors *in vivo,* as by treatment that prevents cell proliferation into tumors. Such treatments are well known in the art.

Cells include:
1. Isolated expanded non-embryonic stem, non-germ cells, the cells having undergone at least 10-40 cell doublings in culture, wherein the cells express oct4, are not transformed, and have a normal karyotype.
2. The non-embryonic stem, non-germ cells of 1 above that further express one or more of telomerase, rex-1, rox-1, or sox-2.
3. The non-embryonic stem, non-germ cells of 1 above that can differentiate into at least one cell type of at least two of the endodermal, ectodermal, and mesodermal embryonic lineages.
4. The non-embryonic stem, non-germ cells of 3 above that further express one or more of telomerase, rex-1, rox-1, or sox-2.
5. The non-embryonic stem, non-germ cells of 3 above that can differentiate into at least one cell type of each of the endodermal, ectodermal, and mesodermal embryonic lineages.
6. The non-embryonic stem, non-germ cells of 5 above that further express one or more of telomerase, rex-1, rox-1, or sox-2.
7. Isolated expanded non-embryonic stem, non-germ cells that are obtained by culture of non-embryonic, non-germ tissue, the cells having undergone at least 40 cell doublings in culture, wherein the cells are not transformed and have a normal karyotype.
8. The non-embryonic stem, non-germ cells of 7 above that express one or more of oct4, telomerase, rex-1, rox-1, or sox-2.
9. The non-embryonic stem, non-germ cells of 7 above that can differentiate into at least one cell type of at least two of the endodermal, ectodermal, and mesodermal embryonic lineages.
10. The non-embryonic stem, non-germ cells of 9 above that express one or more of oct4, telomerase, rex-1, rox-1, or sox-2.
11. The non-embryonic stem, non-germ cells of 9 above that can differentiate into at least one cell type of each of the endodermal, ectodermal, and mesodermal embryonic lineages.
12. The non-embryonic stem, non-germ cells of 11 above that express one or more of oct4, telomerase, rex-1, rox-1, or sox-2.
13. Isolated expanded non-embryonic stem, non-germ cells, the cells having undergone at least 10-40 cell doublings in culture, wherein the cells express telomerase, are not transformed, and have a normal karyotype.
14. The non-embryonic stem, non-germ cells of 13 above that further express one or more of oct4, rex-1, rox-1, or sox-2.
15. The non-embryonic stem, non-germ cells of 13 above that can differentiate into at least one cell type of at least two of the endodermal, ectodermal, and mesodermal embryonic lineages.
16. The non-embryonic stem, non-germ cells of 15 above that further express one or more of oct4, rex-1, rox-1, or sox-2.
17. The non-embryonic stem, non-germ cells of 15 above that can differentiate into at least one cell type of each of the endodermal, ectodermal, and mesodermal embryonic lineages.
18. The non-embryonic stem, non-germ cells of 17 above that further express one or more of oct4, rex-1, rox-1, or sox-2.
19. Isolated expanded non-embryonic stem, non-germ cells that can differentiate into at least one cell type of at least two of the endodermal, ectodermal, and mesodermal embryonic lineages, said cells having undergone at least 10-40 cell doublings in culture.
20. The non-embryonic stem, non-germ cells of 19 above that express one or more of oct4, telomerase, rex-1, rox-1, or sox-2.
21. The non-embryonic stem, non-germ cells of 19 above that can differentiate into at least one cell type of each of the endodermal, ectodermal, and mesodermal embryonic lineages.
22. The non-embryonic stem, non-germ cells of 21 above that express one or more of oct4, telomerase, rex-1, rox-1, or sox-2.

The subject may be human.

The cells that express and/or secrete one or more pro-angiogenic factors can be used in drug discovery methods to screen for an agent that modulates the ability of the cells to express and/or secrete one or more pro-angiogenic factors so as to be able to provide angiogenesis. Such agents include, but are not limited to, small organic molecules, antisense nucleic acids, siRNA, DNA aptamers, peptides, antibodies, non-antibody proteins, cytokines, chemokines, and chemo-attractants.

Potency may be enhanced by exposing the cells to a combination of TNF-α, IL-1β, and IFN-γ. Any of these components could be used individually. Other pro-inflammatory molecules could be used, including, but not limited to, other interleukins or interferons such as Il1-α, IL-6, TGF-b, GM-CSF, IL11, IL12, IL17, IL18, IL8, toll-like receptor ligands including LPS, Poly(1:C), CPGN-ODN, and zymosan. Potency may be enhanced by exposing the cells to latanoprost, a prostaglandin F analog. The cells can be exposed to prostaglandin F, any other prostaglandin F2 alpha receptor analog, E-type prostaglandins or analogs.

Because the angiogenic effects described in this application can be caused by secreted factors, not only the cells, but also conditioned medium (or extracts thereof) produced from culturing the cells, are useful to achieve the effects. Such medium would contain the secreted factors and, therefore, could be used instead of the cells or added to the cells. So, where cells can be used, it should be understood that conditioned medium (or extracts thereof) would also be effective and could be substituted or added.

In view of the property of the cells to achieve the angiogenic effects, cell banks can be established containing cells that are selected for having a desired potency to express and secrete one or more pro-angiogenic factors so as to provide angiogenesis. Accordingly, also disclosed is assaying cells for the ability to express and/or secrete one or more pro-angiogenic factors and banking the cells having a desired potency. The bank can provide a source for making a pharmaceutical composition to administer to a subject. Cells can be used directly from the bank or expanded prior to use. Especially in the case that the cells are subjected to further expansion, after expansion it is desirable to validate that the cells still have the desired potency. Banks allow the "off the shelf" use of cells that are allogeneic to the subj ect.

Accordingly, also disclosed are diagnostic procedures conducted prior to administering the cells to a subject. The procedures include assessing the potency of the cells to express and/or secrete one or more pro-angiogenic factors so as to be able to provide angiogenesis. The cells may be taken from a cell bank and used directly or expanded prior to administration. In either case, the cells could be assessed for the desired potency. Especially in the case that the cells are subjected to further expansion, after expansion it is desirable to validate that the cells still have the desired potency. Or the cells can be derived from the subject and expanded prior to administration. In this case, as well, the cells could be assessed for the desired potency prior to administration back to the subject (autologous).

Although the cells that are selected for expression of the one or more pro-angiogenic factors are necessarily assayed during the selection procedure, it may be preferable and prudent to again assay the cells prior to administration to a subject for treatment to confirm that the cells still express desired levels of the factors. This is particularly preferable where the expressor cells have been expanded or have been stored for any length of time, such as in a cell bank, where cells are most likely frozen during storage.

With respect to methods of treatment with cells expressing/secreting one or more pro-angiogenic factors, between the original isolation of the cells and the administration to a subject, there may be multiple (i.e., sequential) assays for factor(s) expression. This is to confirm that the cells still express/secrete the one or more pro-angiogenic factors after manipulations that occur within this time frame. For example, an assay may be performed after each expansion of the cells. If cells are stored in a cell bank, they may be assayed after being released from storage. If they are frozen, they may be assayed after thawing. If the cells from a cell bank are expanded, they may be assayed after expansion. Preferably, a portion of the final cell product (i.e., the cell preparation that is physically administered to the subject) may be assayed.

The disclosure further includes post-treatment diagnostic assays, following administration of the cells, to assess efficacy. The diagnostic assays include, but are not limited to, analysis of angiogenesis by clinical symptoms, morphologically (e.g., presence of vessels) or by one or more biomarkers of angiogenesis.

The disclosure is also directed to methods for establishing the dosage of the cells by assessing the potency of the cells to express and/or secrete one or more pro-angiogenic factors so as to provide angiogenesis. In this case, the potency would be determined and the dosage adjusted accordingly.

Potency can be assessed by measuring the amounts of the factors themselves. It can also be assessed by assaying effects that the factors provide, such as *in vivo* or *in vitro* angiogenesis.

The disclosure is also directed to compositions comprising a population of the cells having a desired potency, and, particularly the expression and/or secretion of desired amounts of one or more pro-angiogenic factors. Such populations may be found as pharmaceutical compositions suitable for administration to a subject and/or in cell banks from which cells can be used directly for administration to a subject or expanded prior to administration. The cells may have enhanced (increased) potency compared to the previous (parent) cell population. Parent cells are as defined herein. Enhancement can be by selection of natural expressors or by external factors acting on the cells.

The methods and compositions of the disclosure are useful for treating any disease in which angiogenesis is beneficial. This includes, but is not limited to, any ischemic condition, for example, acute myocardial infarction, chronic heart failure, peripheral vascular disease, stroke, chronic total occlusion, renal ischemia, and acute kidney injury.

For these treatments, one would administer the cells expressing the one or more pro-angiogenic factors. Such cells could have been assessed for the amount of the factor(s) that they express and/or secrete and selected for desired amounts of expression and/or secretion of the factor(s).

It is understood that for treatment of any of the above diseases, it may be expedient to use such cells; that is, one that has been assessed for factor(s) expression and/or secretion and selected for a desired level of expression and/or secretion prior to administration for treatment of the condition.

### BRIEF DESCRIPTION OF THE FIGURES

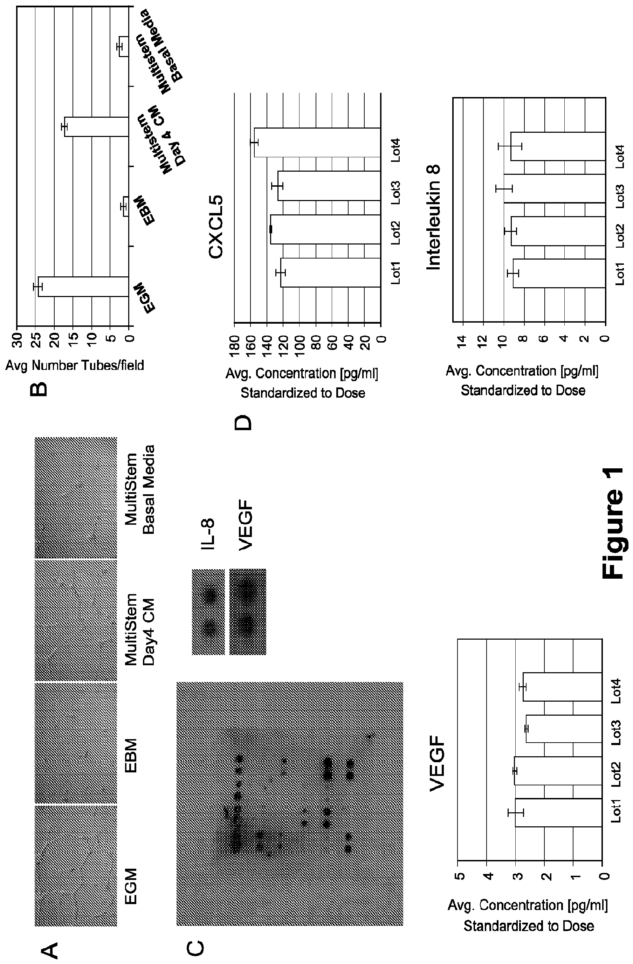

Figure 1 - MultiStem induces angiogenesis *in vitro* and secretes multiple pro-angiogenic factors (A). Photographs of *in vitro* angiogenesis induced by MultiStem conditioned media (CM) with cultured HUVECs. (**B**) Average number of tubes formed per field in each condition (**C**) Angiogenesis antibody array incubated with MultiStem day 4 conditioned media. VEGF, IL-8 and CXLC5 are secreted by MultiStem. CXCL5 (**D**) VEGF (**E**) and IL-8 (**F**) protein concentrations in 3 day spent media from four separate cultures illustrate that MultiStem consistently express these proteins under standard culturing conditions.

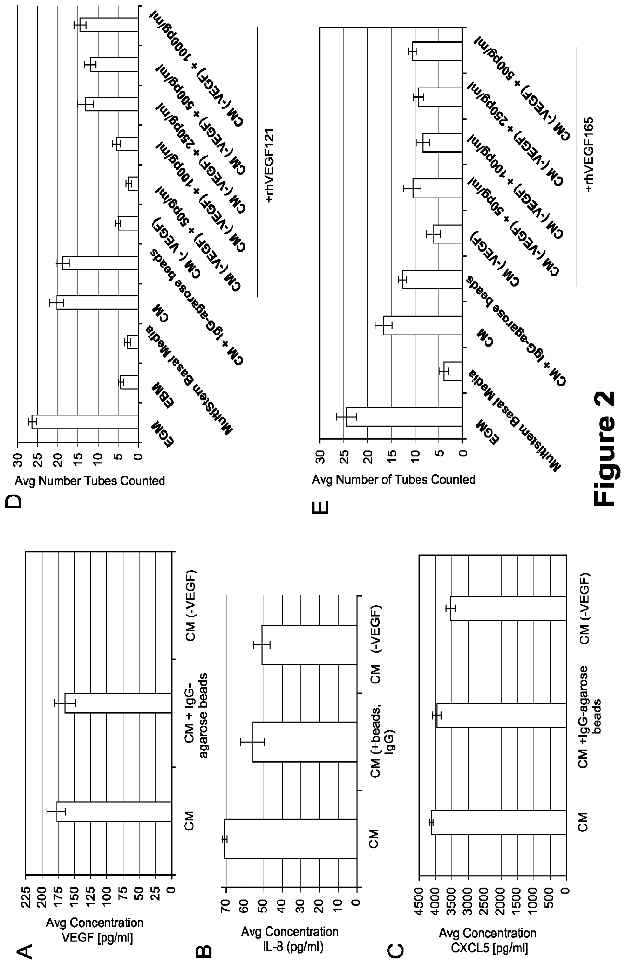

Figure 2 - VEGF is required for MultiStem induced angiogenesis. Removal of VEGF from conditioned media prevents angiogenesis (**A**) complete VEGF immunodepletion and antibody specificity while IL-8 (**B**) and CXCL5 (**C**) levels are unaffected. (**D**,**E**) Immunodepletion of VEGF reduces angiogenesis induced by MultiStem conditioned media. Addition of at least 250 pg/ml of VEGF165 or 50 pg/ml of VEGF121 is required to restore some level of angiogenesis although neither completely restored activity.

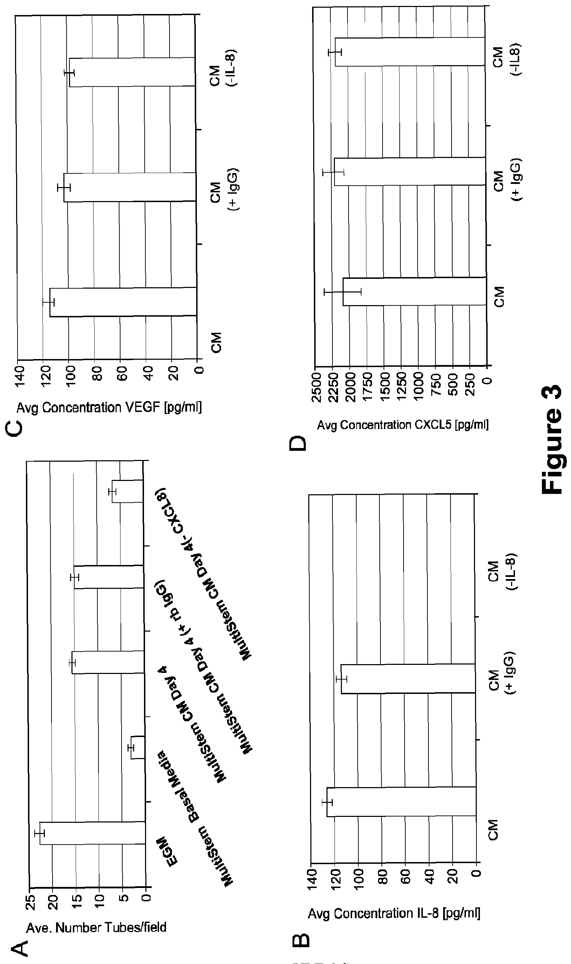

Figure 3 - IL-8 is necessary MultiStem induced angiogenesis. Immunodepletion of IL-8 from the conditioned media reduced angiogenesis but addition of IL-8 to basal media was insufficient to induce angiogenesis. (**A**) HUVECS were incubated for 18hrs with (a) endothelial growth factor media (EGM), (b) serum-free basal MultiStem Media, (c) 4-day, serum-free MultiStem CM, (d) rabbit IgG isotype control, and (e) 4-day, serum-free MultiStem CM immunodepleted of IL-8. (**B**) IL-8 is reduced by immunodepletion (**C**,**D**) VEGF and CXCL5 levels were unchanged.

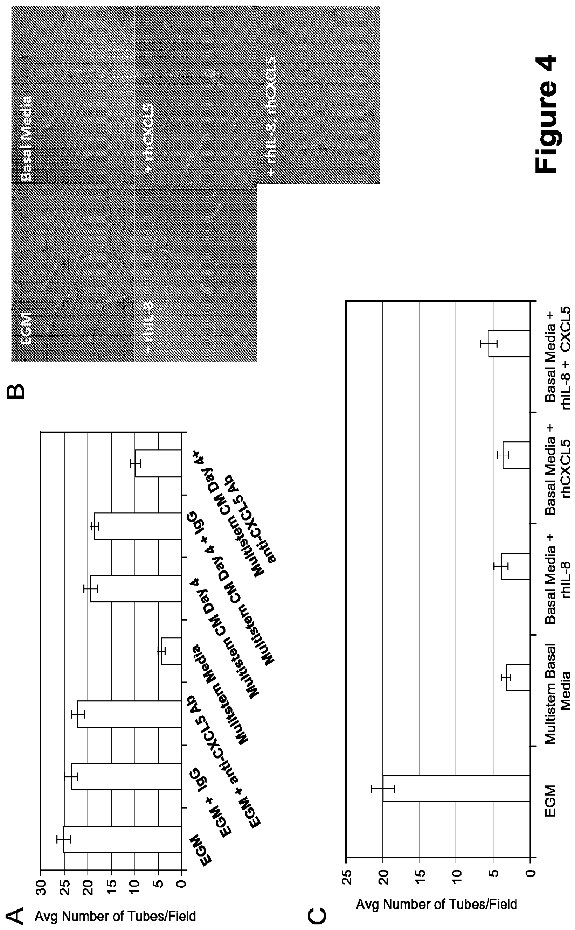

Figure 4 - CXCL5 is required for MultiStem induced angiogenesis. However, IL-8 and CXCL5 are insufficient to initiate angiogenesis. (**A**) HUVECS were incubated for 18hrs with (a) endothelial growth factor media (EGM), (b) EGM + IgG isotype control c) EGM + 10ug/ml CXCL5 neutralizing antibody (d) serum-free basal MultiStem Media (e) 4-day, serum-free MultiStem conditioned media alone (CM) (f) CM + IgG isotype control (g) CM + CXCL5 neutralizing antibody (10ug/ml). (**B**,**C**) Addition of IL-8 (4000 pg/ml) or CXCL5 (150 pg/ml) alone or together to MultiStem Basal Media was insufficient to induced angiogenesis.

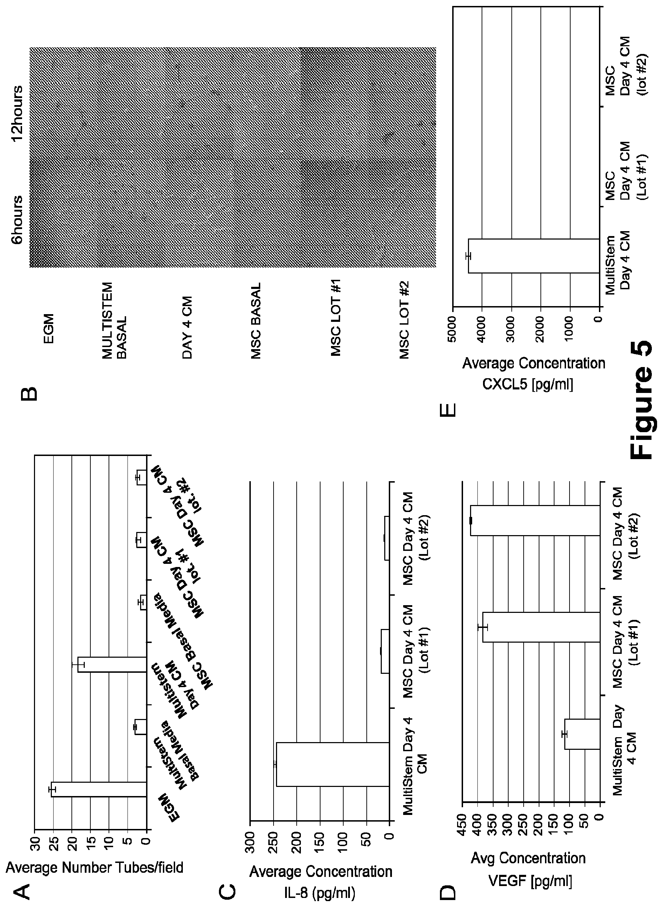

Figure 5 - Unlike MultiStem, MSC do not induce angiogenesis *in vitro.* (**A**) An *in vitro* angiogenesis assay illustrating the difference in the effects of MSC and MultiStem conditioned media (CM) on endothelial cell tube formation (**B**). Photographs from an *in vitro* angiogenesis assay showing the effects of MSC and Multistem CM on endothelial cell tube formation after 6hrs and 24hrs. (**C**-**E**) Concentrations of CXCL5, VEGF, and IL-8 secreted by MSC and MultiStem.

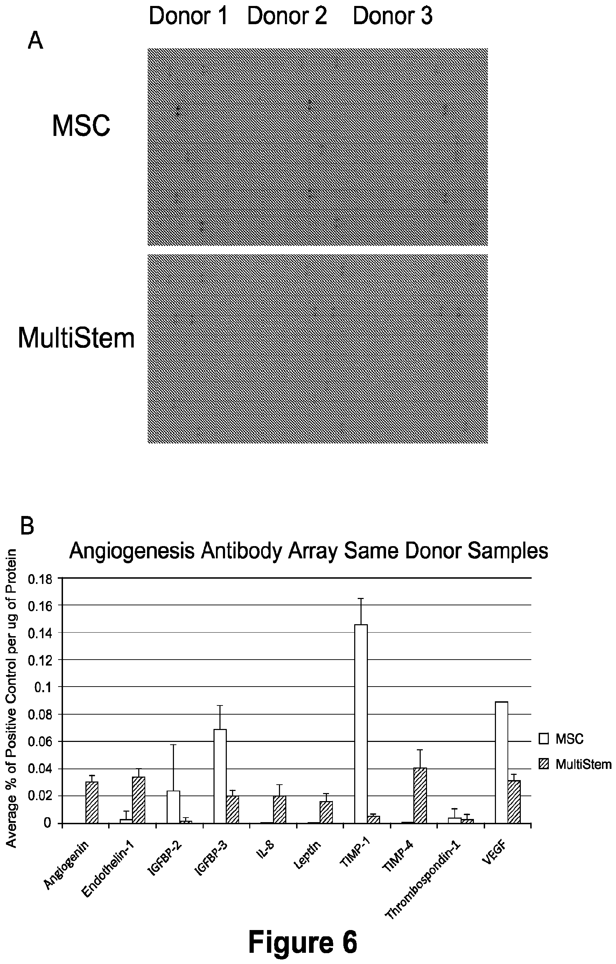

Figure 6 - MultiStem and MSC have distinct secretion profiles. Analysis of conditioned media from MSC and MultiStem derived from the same donor (3 donor sample sets were analyzed) on an angiogenesis specific antibody array. (**A**) Photos of the developed membrane illustrate that the secretion profile of MultiStem is similar to MultiStem from other donors but show significant differences when compared to the secretion profile of MSC, even from the same donor. (**B**) Semi quantitative analysis of the arrays showing distinct secretion profiles of MSC versus MultiStem, including exclusive expression of IL-8 by MultiStem. The data is expressed as the average spot intensity as a percent of positive control, normalized back to the total protein content.

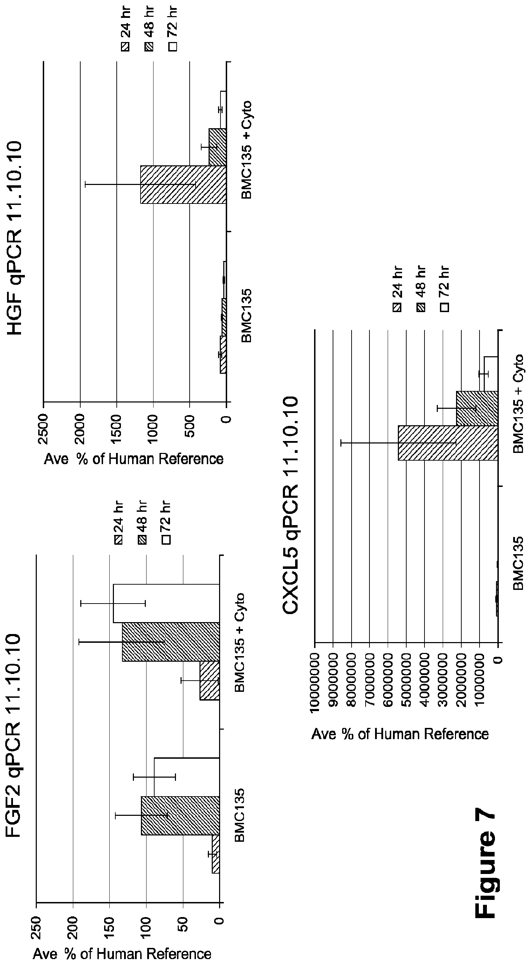

Figure 7 - Treatment of MultiStem with Cytomix increases the expression of pro-angiogenic molecules *in vitro.* MultiStem was grown for three days and then treated with Cytomix (10 ng/mL TNF-α, IL-1β and IFNγ) for 24, 48, 72 hrs. The cells were subsequently collected for RT-PCR analysis for pro-angiogenic gene expression. CXCL5, FGF2 and HGF gene expression were all increased over baseline with cytomix treatment. Additionally, IL-8 is also increased in these conditions

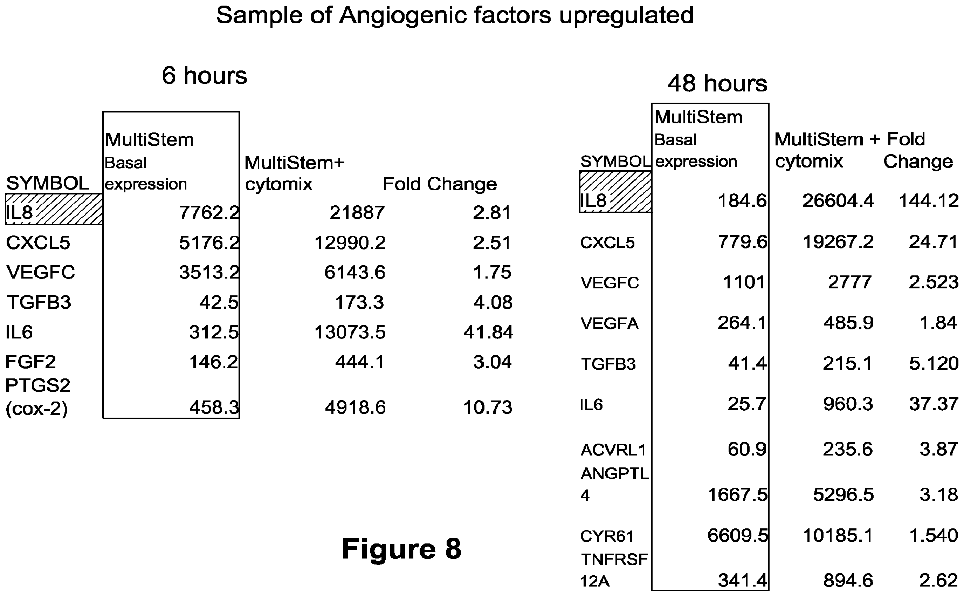

Figure 8 - Microarray analysis also shows an upregulation of the expression of angiogenic genes in MultiStem treated with Cytomix (48hrs). The figure shows a sample of angiogenic factors that are regulated. Microarray analysis shows an increase in pro-angiogenic factors in MultiStem treated with Cytomix for 6 or 48 hours. RNA from MultiStem treated with cytomix (n=6 per time point) or untreated MultiStem (n=6 per time point) was analyzed on an Illumina microarray chip (HumanHT-12_V4). Two MultiStem banks were examined. This figure gives examples of the fold increase for a sample of the pro-angiogenic genes upregulated. Further confirmation by qPCR is required and is currently in process.

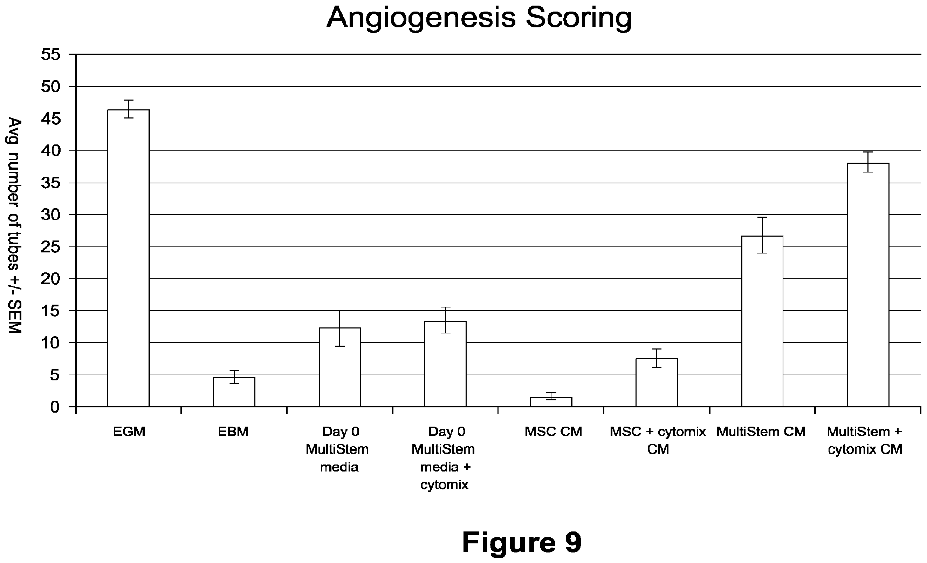

Figure 9 - Cytomix treatment increases the angiogenic potential of MultiStem in the HUVEC tube formation assay. The figure shows angiogenesis scoring. Treatment of MultiStem with Cytomix increases angiogenesis in the HUVEC tube formation assay. Serum-free conditioned media collected from cells after three days shows that while conditioned media from untreated MultiStem gave robust HUVEC tube formation, treatment of the cells with Cytomix resulted in an increase of angiogenic potential. Serum-free conditioned media from Lonza MSCs did not induce significant HUVEC tube formation. Treatment of MSCs only slightly increased the angiogenic potential. EGM= endothelial growth media (positive control). EBM= serum-free basal endothelial media (negative control).

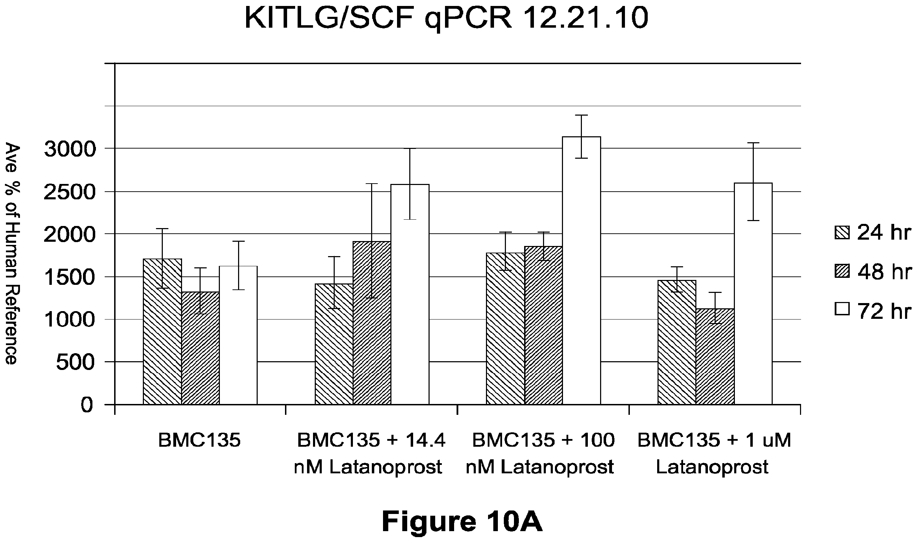

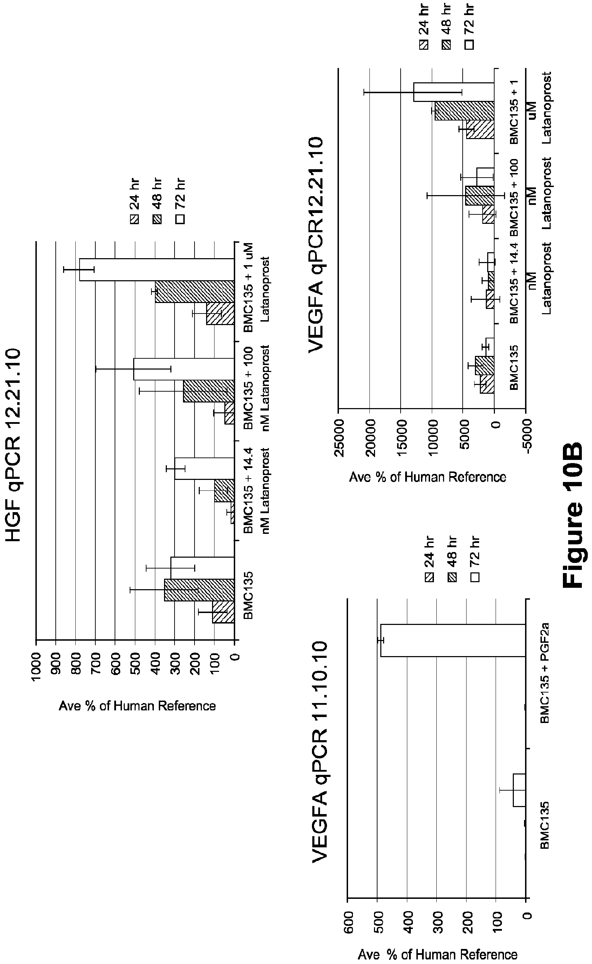

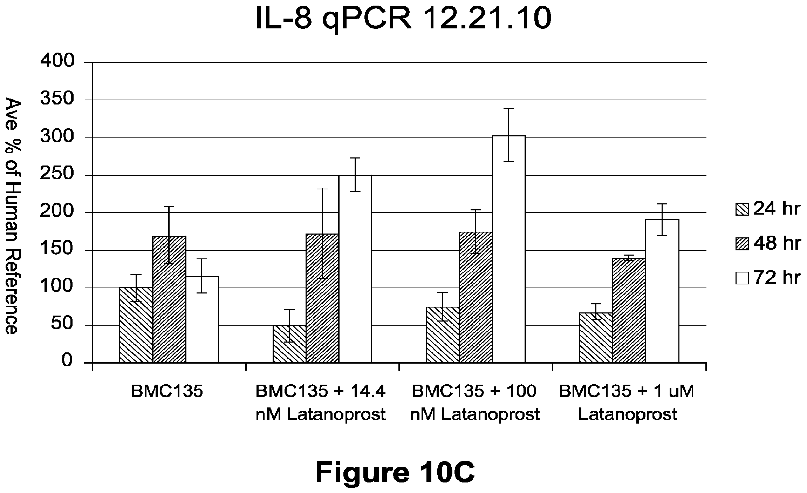

Figures 10A-C - Pre-treatment of MultiStem with prostaglandin F or latanoprost (prostaglandin F agonist) increases expression of the angiogenic factors by MultiStem *in vitro.* Treatment of MultiStem with a prostaglandin F analog, latanoprost, also increased the expression of pro-angiogenic factors. MultiStem was treated with a dose range of Latanoprost for 24, 48 or 72 hours. Gene expression of pro-angiogenic factors was then analyzed by RT-PCR. Gene expression of KITLG (A), HGF and VEGF (B), and Il-8 (C) were all increased. The biologic, prostaglandin F, also increased VEGF A levels (B).

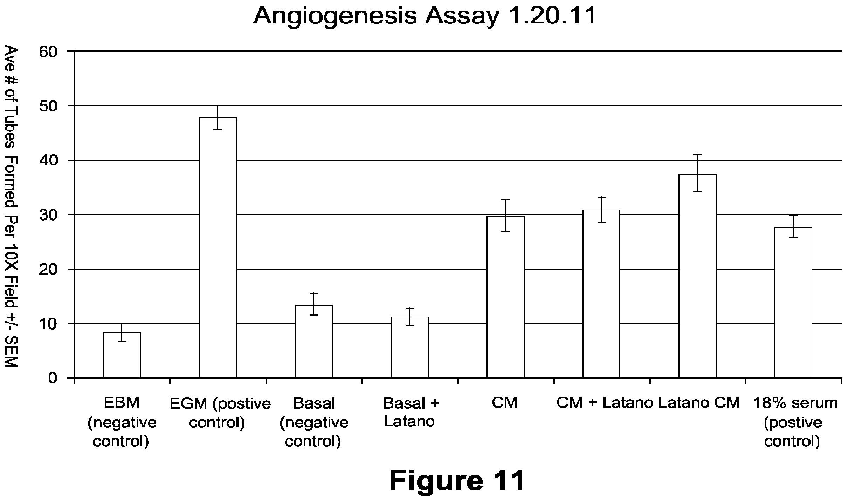

Figure 11 - HUVEC tube formation assay to test angiogenic potential of latanoprost (1uM)-treated MultiStem. HUVEC tube formation increased modestly with conditioned media from latanoprost treated cells compared to conditioned media from untreated cells. Serum-free media was collected on day three from MultiStem cultured alone or in the presence of latanoprost (1 uM). Basal media or basal media with added latanoprost did no induce significant tube formation. In contrast, conditioned media from untreated cells alone or with latanoprost (1 uM) added to the media after collection, induced angiogenesis to equal levels. Serum-free conditioned media collected from cells treated for three day with latanoprost increased the angiogenic potential modestly, as measure by this *in vitro* assay. EGM and serum containing MultiStem media served as positive controls. EBM and Basal serum-free media were the negative controls.

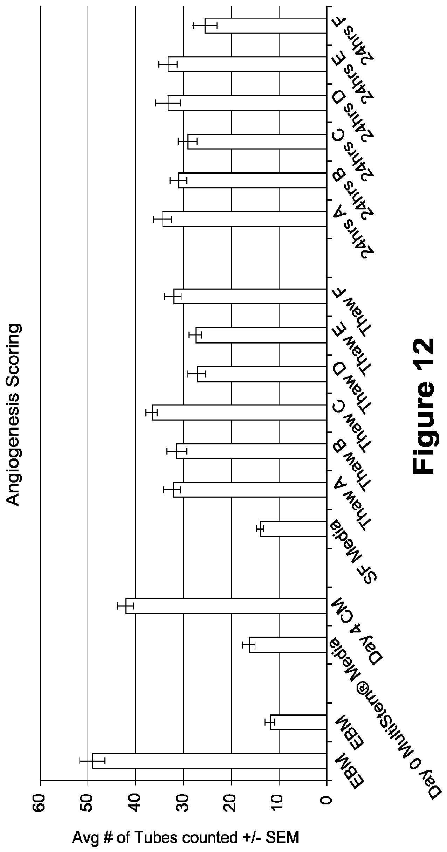

Figure 12 - *In vitro* angiogenesis analysis can be utilized to examine the potency of different cell lots and processing protocols. Measurement of the angiogenic potential by using the HUVEC tube formation assay can be used to assess the function potency of cells from different cell lots (thaw a-f) or different processing conditioned (thaw-f versus 24 hr A-F).

### DETAILED DESCRIPTION OF THE INVENTION

The section headings are used herein for organizational purposes only and are not to be construed as in any way limiting the subject matter described.

The methods and techniques of the present application are generally performed according to conventional methods well-known in the art and as described in various general and more specific references that are cited and discussed throughout the present specification unless otherwise indicated. See, e.g., Sambrook et al., Molecular Cloning: A Laboratory Manual, 3rd ed., Cold Spring Harbor Laboratory Press, Cold Spring Harbor, N.Y. (2001) and Ausubel et al., Current Protocols in Molecular Biology, Greene Publishing Associates (1992), and Harlow and Lane, Antibodies: A Laboratory Manual, Cold Spring Harbor Laboratory Press, Cold Spring Harbor, N.Y. (1990).

### Definitions

"A" or "an" means herein one or more than one; at least one. Where the plural form is used herein, it generally includes the singular.

A "cell bank" is industry nomenclature for cells that have been grown and stored for future use. Cells may be stored in aliquots. They can be used directly out of storage or may be expanded after storage. This is a convenience so that there are "off the shelf" cells available for administration. The cells may already be stored in a pharmaceutically-acceptable excipient so they may be directly administered or they may be mixed with an appropriate excipient when they are released from storage. Cells may be frozen or otherwise stored in a form to preserve viability. Cell banks may be created in which the cells have been selected for enhanced expression of one or more pro-angiogenic factors. Following release from storage, and prior to administration to the subject, it may be preferable to again assay the cells for potency, i.e., level of expression of one or more pro-angiogenic factors. This can be done using any of the assays, direct or indirect, described in this application or otherwise known in the art. Then cells having the desired potency can then be administered to the subject for treatment. Banks can be made using cells derived from the individual to be treated (from their pre-natal tissues such as placenta, umbilical cord blood, or umbilical cord matrix or expanded from the individual at any time after birth). Or banks can contain cells for allogeneic uses.

"Co-administer" means to administer in conjunction with one another, together, coordinately, including simultaneous or sequential administration of two or more agents.

"Comprising" means, without other limitation, including the referent, necessarily, without any qualification or exclusion on what else may be included. For example, "a composition comprising x and y" encompasses any composition that contains x and y, no matter what other components may be present in the composition. Likewise, "a method comprising the step of x" encompasses any method in which x is carried out, whether x is the only step in the method or it is only one of the steps, no matter how many other steps there may be and no matter how simple or complex x is in comparison to them. "Comprised of and similar phrases using words of the root "comprise" are used herein as synonyms of "comprising" and have the same meaning.

"Comprised of" is a synonym of "comprising" (see above).

"Conditioned cell culture medium" is a term well-known in the art and refers to medium in which cells have been grown. Herein this means that the cells are grown for a sufficient time to secrete the factors that are effective to achieve any of the results described in this application, including providing angiogenesis or providing one or more pro-angiogenic factors.

Conditioned cell culture medium refers to medium in which cells have been cultured so as to secrete factors into the medium. Cells can be grown through a sufficient number of cell divisions so as to produce effective amounts of such factors so that the medium has the effects, including providing angiogenesis or providing one or more pro-angiogenic factors. Cells are removed from the medium by any of the known methods in the art, including, but not limited to, centrifugation, filtration, immunodepletion (e.g., via tagged antibodies and magnetic columns), and FACS sorting.

"EC cells" were discovered from analysis of a type of cancer called a teratocarcinoma. In 1964, researchers noted that a single cell in teratocarcinomas could be isolated and remain undifferentiated in culture. This type of stem cell became known as an embryonic carcinoma cell (EC cell).

"Effective amount" generally means an amount which provides the desired local or systemic effect that results from providing angiogenesis. For example, an effective amount is an amount sufficient to effectuate a beneficial or desired clinical result. The effective amount can be provided all at once in a single administration or in fractional amounts that provide the effective amount in several administrations. The precise determination of what would be considered an effective amount may be based on factors individual to each subject, including their size, age, injury, and/or disease or injury being treated, and amount of time since the injury occurred or the disease began. One skilled in the art will be able to determine the effective amount for a given subject based on these considerations which are routine in the art. As used herein, with respect to treatment, "effective dose" means the same as "effective amount."

"Effective route" generally means a route which provides for delivery of an agent to a desired compartment, system, or location. For example, an effective route is one through which an agent can be administered to provide at the desired site of action an amount of the agent sufficient to effectuate a beneficial or desired clinical result.

"Embryonic Stem Cells (ESC)" are well known in the art and have been prepared from many different mammalian species. ESCs are described herein for reference only as they not covered by the claims. Embryonic stem cells are stem cells derived from the inner cell mass of an early stage embryo known as a blastocyst. They are able to differentiate into all derivatives of the three primary germ layers: ectoderm, endoderm, and mesoderm. These include each of the more than 220 cell types in the adult body. The ES cells can become any tissue in the body, excluding placenta. Only the morula's cells are totipotent, able to become all tissues and a placenta. Some cells similar to ESCs may be produced by nuclear transfer of a somatic cell nucleus into an enucleated fertilized egg.

Use of the term "includes" is not intended to be limiting.

"Increase" or "increasing" means to induce a biological event entirely or to increase the degree of the event.

"Induced pluripotent stem cells (IPSC or IPS cells)" are somatic cells that have been reprogrammed, for example, by introducing exogenous genes that confer on the somatic cell a less differentiated phenotype. These cells can then be induced to differentiate into less differentiated progeny. IPS cells have been derived using modifications of an approach originally discovered in 2006 (Yamanaka, S. et al., Cell Stem Cell, 1:39-49 (2007)). For example, in one instance, to create IPS cells, scientists started with skin cells that were then modified by a standard laboratory technique using retroviruses to insert genes into the cellular DNA. In one instance, the inserted genes were Oct4, Sox2, Lif4, and c-myc, known to act together as natural regulators to keep cells in an embryonic stem cell-like state. These cells have been described in the literature. See, for example, Wernig et al., PNAS, 105:5856-5861 (2008); Jaenisch et al., Cell, 132:567-582 (2008); Hanna et al., Cell, 133:250-264 (2008); and Brambrink et al., Cell Stem Cell, 2:151-159 (2008). These references teach IPSCs and methods for producing them. It is also possible that such cells can be created by specific culture conditions (exposure to specific agents).

The term "isolated" refers to a cell or cells which are not associated with one or more cells or one or more cellular components that are associated with the cell or cells *in vivo.* An "enriched population" means a relative increase in numbers of a desired cell relative to one or more other cell types *in vivo* or in primary culture.

However, as used herein, the term "isolated" does not indicate the presence of only stem cells. Rather, the term "isolated" indicates that the cells are removed from their natural tissue environment and are present at a higher concentration as compared to the normal tissue environment. Accordingly, an "isolated" cell population may further include cell types in addition to stem cells and may include additional tissue components. This also can be expressed in terms of cell doublings, for example. A cell may have undergone 10, 20, 30, 40 or more doublings *in vitro* or *ex vivo* so that it is enriched compared to its original numbers *in vivo* or in its original tissue environment (e.g., bone marrow, peripheral blood, placenta, umbilical cord, umbilical cord blood, adipose tissue, etc.).

"MAPC" is an acronym for "multipotent adult progenitor cell." It refers to a cell that is not an embryonic stem cell or germ cell but has some characteristics of these. MAPC can be characterized in a number of alternative descriptions, each of which conferred novelty to the cells when they were discovered. They can, therefore, be characterized by one or more of those descriptions. First, they have extended replicative capacity in culture without being transformed (tumorigenic) and with a normal karyotype. Second, they may give rise to cell progeny of more than one germ layer, such as two or all three germ layers (i.e., endoderm, mesoderm and ectoderm) upon differentiation. Third, although they are not embryonic stem cells or germ cells, they may express markers of these primitive cell types so that MAPCs may express one or more of Oct 3/4 (i.e., Oct 3A), rex-1, and rox-1. They may also express one or more of sox-2 and SSEA-4. Fourth, like a stem cell, they may self-renew, that is, have an extended replication capacity without being transformed. This means that these cells express telomerase (i.e., have telomerase activity). Accordingly, the cell type that was designated "MAPC" may be characterized by alternative basic characteristics that describe the cell via some of its novel properties.

The term "adult" in MAPC is non-restrictive. It refers to a non-embryonic somatic cell. MAPCs are karyotypically normal and do not form teratomas *in vivo.* This acronym was first used in U.S. Patent No. 7,015,037 to describe a pluripotent cell isolated from bone marrow. However, cells with pluripotential markers and/or differentiation potential have been discovered subsequently and, may be equivalent to those cells first designated "MAPC." Essential descriptions of the MAPC type of cell are provided in the Summary of the Invention above.

MAPC represents a more primitive progenitor cell population than MSC (Verfaillie, C.M., Trends Cell Biol 12:502-8 (2002), Jahagirdar, B.N., et al., Exp Hematol, 29:543-56 (2001); Reyes, M. and C.M. Verfaillie, Ann N YAcad Sci, 938:231-233 (2001); Jiang, Y. et al., Exp Hematol, 30896-904 (2002); and (Jiang, Y. et al., Nature, 418:41-9. (2002)).

The term "MultiStem®" is the trade name for a cell preparation based on the MAPCs of U.S. Patent No. 7,015,037, i.e., a non-embryonic stem, non-germ cell as described above. MultiStem® is prepared according to cell culture methods disclosed in this patent application, particularly, lower oxygen and higher serum.

"Pharmaceutically-acceptable carrier" is any pharmaceutically-acceptable medium for the cells used. Such a medium may retain isotonicity, cell metabolism, pH, and the like. It is compatible with administration to a subject *in vivo,* and can be used, therefore, for cell delivery and treatment.

The term "potency" refers to the degree of effectiveness of the cells (or conditioned medium from the cells) to achieve the various effects described in this application. Accordingly, potency refers to the effect at various levels, including, but not limited to, (1) providing angiogenesis, (2) expressing and/or secreting one or more pro-angiogenic factors, or (3) treating a clinical symptom associated with inadequate angiogenesis so as to reduce (including prevent) the symptom.

"Primordial embryonic germ cells" (PG or EG cells) can be cultured and stimulated to produce many less differentiated cell types. PG or EG cells are described herein for reference only as they are not covered by the claims.

"Progenitor cells" are cells produced during differentiation of a stem cell that have some, but not all, of the characteristics of their terminally-differentiated progeny. Defined progenitor cells, such as "cardiac progenitor cells," are committed to a lineage, but not to a specific or terminally differentiated cell type. The term "progenitor" as used in the acronym "MAPC" does not limit these cells to a particular lineage. A progenitor cell can form a progeny cell that is more highly differentiated than the progenitor cell.

The term "reduce" as used herein means to prevent as well as decrease. In the context of treatment, to "reduce" is to either prevent or ameliorate one or more clinical symptoms. A clinical symptom is one (or more) that has or will have, if left untreated, a negative impact on the quality of life (health) of the subject. This also applies to the underlying biological effects, the end result of which would be to ameliorate the deleterious effects of inadequate angiogenesis.

"Selecting" a cell with a desired level of potency (e.g., for expressing and/or secreting one or more pro-angiogenic factors) can mean identifying (as by assay), isolating, and expanding a cell. This could create a population that has a higher potency than the parent call population from which the cell was isolated. The "parent" cell population refers to the parent cells from which the selected cells divided. "Parent" refers to an actual P1 → F1 relationship (i.e., a progeny cell). So if cell X is isolated from a mixed population of cells X and Y, in which X is an expressor and Y is not, one would not classify a mere isolate of X as having enhanced expression. But, if a progeny cell of X is a higher expressor, one would classify the progeny cell as having enhanced expression.

To select a cell that expresses the one or more pro-angiogenic factors, would include both an assay to determine if there is expression/secretion of the one or more pro-angiogenic factors and would also include obtaining the expressor cell. The expressor cell may naturally express the one or more pro-angiogenic factors in that the cell does not express the factor(s) by recombinant means. But an expressor may be improved by being incubated with or exposed to an agent that increases factor expression. The cell population from which the expressor cell is selected may not be known to express the one or more pro-angiogenic factors prior to conducting the assay.

Selection could be from cells in a tissue. For example, in this case, cells would be isolated from a desired tissue, expanded in culture, selected for expression/secretion of one or more pro-angiogenic factors, and the selected cells further expanded.

Selection could also be from cells *ex vivo*, such as cells in culture. In this case, one or more of the cells in culture would be assayed for expression/secretion of one or more pro-angiogenic factors and the cells obtained that express/secrete one or more pro-angiogenic factors could be further expanded.

Cells could also be selected for enhanced expression/secretion of one or more pro-angiogenic factors. In this case, the cell population from which the enhanced expresser is obtained may already express/secrete the one or more pro-angiogenic factors. Enhanced expression/secretion means a higher average amount (expression and/or secretion) of one or more pro-angiogenic factors per cell than in the parent expressor population.

The parent population from which the higher expressor is selected may be substantially homogeneous (the same cell type). One way to obtain a higher expresser from this population is to create single cells or cell pools and assay those cells or cell pools for expression/secretion of one or more pro-angiogenic factors to obtain clones that naturally express/secrete enhanced levels of one or more pro-angiogenic factors (as opposed to treating the cells with an inducer of one or more pro-angiogenic factors) and then expanding those cells that are naturally higher expressors.

However, cells may be treated with one or more agents that will enhance factor expression of the endogenous cellular gene for the factor. Thus, substantially homogeneous populations may be treated to enhance expression.

If the population is not substantially homogeneous, then, it is preferable that the parental cell population to be treated contains at least 100 of the expressor cell type in which enhanced expression is sought, more preferably at least 1,000 of the cells, and still more preferably, at least 10,000 of the cells. Following treatment, this sub-population can be recovered from the heterogeneous population by known cell selection techniques and further expanded if desired.

Thus, desired levels of factor expression may be those that are higher than the levels in a given preceding population. For example, cells that are put into primary culture from a tissue and expanded and isolated by culture conditions that are not specifically designed to promote factor expression, may provide a parent population. Such a parent population can be treated to enhance the average factor expression per cell or screened for a cell or cells within the population that express higher levels without deliberate treatment. Such cells can be expanded then to provide a population with a higher (desired) expression.

"Self-renewal" refers to the ability to produce replicate daughter stem cells having differentiation potential that is identical to those from which they arose. A similar term used in this context is "proliferation."

"Stem cell" means a cell that can undergo self-renewal (i.e., progeny with the same differentiation potential) and also produce progeny cells that are more restricted in differentiation potential. Within the context of the invention, a stem cell would also encompass a more differentiated cell that has de-differentiated, for example, by introduction of specific transcription factors, or by culture under specific conditions. For reference, outside the context of the invention de-differentiation may also occur by nuclear transfer or by fusion with a more primitive stem cell. See, for example, Wilmut et al., Nature, 385:810-813 (1997); Ying et al., Nature, 416:545-548 (2002); Guan et al., Nature, 440:1199-1203 (2006); Takahashi et al., Cell, 126:663-676 (2006); Okita et al., Nature, 448:313-317 (2007); and Takahashi et al., Cell, 131:861-872 (2007).

Dedifferentiation may also be caused by the administration of certain compounds or exposure to a physical environment *in vitro* or *in vivo* that would cause the dedifferentiation. Stem cells also may be derived from abnormal tissue, such as a teratocarcinoma and some other sources such as embryoid bodies (although these can be considered embryonic stem cells in that they are derived from embryonic tissue, although not directly from the inner cell mass). Stem cells may also be produced by introducing genes associated with stem cell function into a non-stem cell, such as an induced pluripotent stem cell.

"Subject" means a vertebrate, such as a mammal, such as a human. Mammals include, but are not limited to, humans, dogs, cats, horses, cows, and pigs.

The term "therapeutically effective amount" refers to the amount of an agent determined to produce any therapeutic response in a mammal. For example, effective therapeutic agents may prolong the survivability of the patient, and/or inhibit overt clinical symptoms. Treatments that are therapeutically effective within the meaning of the term as used herein, include treatments that improve a subject's quality of life even if they do not improve the disease outcome per se. Such therapeutically effective amounts are readily ascertained by one of ordinary skill in the art. Thus, to "treat" means to deliver such an amount. Thus, treating can prevent or ameliorate pathological symptoms of inadequate angiogenesis.

"Treat," "treating," or "treatment" are used broadly in relation to the invention and each such term encompasses, among others, preventing, ameliorating, inhibiting, or curing a deficiency, dysfunction, disease, or other deleterious process, including those that interfere with and/or result from a therapy.

"Validate" means to confirm. In the context of the disclosure, one confirms that a cell is an expressor with a desired potency. This is so that one can then use that cell (in treatment, banking, drug screening, etc.) with a reasonable expectation of efficacy. Accordingly, to validate means to confirm that the cells, having been originally found to have/established as having pro-angiogenic activity, in fact, retain that activity. Thus, validation is a verification event in a two-event process involving the original determination and the follow-up determination. The second event is referred to herein as "validation."

### Stem Cells

The present invention can be practiced, using stem cells only as defined in the claims, which may be of vertebrate species, such as humans, non-human primates, domestic animals, livestock, and other non-human mammals. Types of stem cells (those which are embryonic stem cells and germ cells fall outside the scope of the claims and are marked "for reference" accordingly) are described below.

### Embryonic Stem Cells (for reference)

The most well studied stem cell is the embryonic stem cell (ESC) as it has unlimited self-renewal and multipotent differentiation potential. These cells are derived from the inner cell mass of the blastocyst or can be derived from the primordial germ cells of a post-implantation embryo (embryonal germ cells or EG cells). ES and EG cells have been derived, first from mouse, and later, from many different animals, and more recently, also from non-human primates and humans. When introduced into mouse blastocysts or blastocysts of other animals, ESCs can contribute to all tissues of the animal. ES and EG cells can be identified by positive staining with antibodies against SSEA1 (mouse) and SSEA4 (human). See, for example, U.S. Patent Nos. 5,453,357; 5,656,479; 5,670,372; 5,843,780; 5,874,301; 5,914,268; 6,110,739 6,190,910; 6,200,806; 6,432,711; 6,436,701, 6,500,668; 6,703,279; 6,875,607; 7,029,913; 7,112,437; 7,145,057; 7,153,684; and 7,294,508 which teach embryonic stem cells and methods of making and expanding them. Accordingly, ESCs and methods for isolating and expanding them are well-known in the art.

A number of transcription factors and exogenous cytokines have been identified that influence the potency status of embryonic stem cells *in vivo.* The first transcription factor to be described that is involved in stem cell pluripotency is Oct4. Oct4 belongs to the POU (Pit-Oct-Unc) family of transcription factors and is a DNA binding protein that is able to activate the transcription of genes, containing an octameric sequence called "the octamer motif" within the promoter or enhancer region. Oct4 is expressed at the moment of the cleavage stage of the fertilized zygote until the egg cylinder is formed. The function of Oct3/4 is to repress differentiation inducing genes (i.e., FoxaD3, hCG) and to activate genes promoting pluripotency (FGF4, Utf1, Rex1). Sox2, a member of the high mobility group (HMG) box transcription factors, cooperates with Oct4 to activate transcription of genes expressed in the inner cell mass. It is essential that Oct3/4 expression in embryonic stem cells is maintained between certain levels. Overexpression or downregulation of >50% of Oct4 expression level will alter embryonic stem cell fate, with the formation of primitive endoderm/mesoderm or trophectoderm, respectively. *In vivo*, Oct4 deficient embryos develop to the blastocyst stage, but the inner cell mass cells are not pluripotent. Instead they differentiate along the extraembryonic trophoblast lineage. Sall4, a mammalian Spalt transcription factor, is an upstream regulator of Oct4, and is therefore important to maintain appropriate levels of Oct4 during early phases of embryology. When Sall4 levels fall below a certain threshold, trophectodermal cells will expand ectopically into the inner cell mass. Another transcription factor required for pluripotency is Nanog, named after a celtic tribe "Tir Nan Og": the land of the ever young. *In vivo*, Nanog is expressed from the stage of the compacted morula, is subsequently defined to the inner cell mass and is downregulated by the implantation stage. Downregulation of Nanog may be important to avoid an uncontrolled expansion of pluripotent cells and to allow multilineage differentiation during gastrulation. Nanog null embryos, isolated at day 5.5, consist of a disorganized blastocyst, mainly containing extraembryonic endoderm and no discernable epiblast.

### Non-Embryonic Stem Cells

Stem cells have been identified in most tissues. Perhaps the best characterized is the hematopoietic stem cell (HSC). HSCs are mesoderm-derived cells that can be purified using cell surface markers and functional characteristics. They have been isolated from bone marrow, peripheral blood, cord blood, fetal liver, and yolk sac. They initiate hematopoiesis and generate multiple hematopoietic lineages. When transplanted into lethally-irradiated animals, they can repopulate the erythroid neutrophil-macrophage, megakaryocyte, and lymphoid hematopoietic cell pool. They can also be induced to undergo some self-renewal cell division. See, for example, U.S. Patent Nos. 5,635,387; 5,460,964; 5,677,136; 5,750,397; 5,681,599; and 5,716,827. U.S. Patent No. 5,192,553 reports methods for isolating human neonatal or fetal hematopoietic stem or progenitor cells. U.S. Patent No. 5,716,827 reports human hematopoietic cells that are Thy-1⁺ progenitors, and appropriate growth media to regenerate them *in vitro.* U.S. Patent No. 5,635,387 reports a method and device for culturing human hematopoietic cells and their precursors. U.S. Patent No. 6,015,554 describes a method of reconstituting human lymphoid and dendritic cells. Accordingly, HSCs and methods for isolating and expanding them are well-known in the art.

Another stem cell that is well-known in the art is the neural stem cell (NSC). These cells can proliferate *in vivo* and continuously regenerate at least some neuronal cells. When cultured ex vivo, neural stem cells can be induced to proliferate as well as differentiate into different types of neurons and glial cells. When transplanted into the brain, neural stem cells can engraft and generate neural and glial cells. See, for example, Gage F.H., Science, 287:1433-1438 (2000), Svendsen S.N. et al, Brain Pathology, 9:499-513 (1999), and Okabe S. et al., Mech Development, 59:89-102 (1996). U.S. Patent No. 5,851,832 reports multipotent neural stem cells obtained from brain tissue. U.S. Patent No. 5,766,948 reports producing neuroblasts from newborn cerebral hemispheres. U.S. Patent Nos. 5,564,183 and 5,849,553 report the use of mammalian neural crest stem cells. U.S. Patent No. 6,040,180 reports *in vitro* generation of differentiated neurons from cultures of mammalian multipotential CNS stem cells. WO 98/50526 and WO 99/01159 report generation and isolation of neuroepithelial stem cells, oligodendrocyte-astrocyte precursors, and lineage-restricted neuronal precursors. U.S. Patent No. 5,968,829 reports neural stem cells obtained from embryonic forebrain. Accordingly, neural stem cells and methods for making and expanding them are well-known in the art.

Another stem cell that has been studied extensively in the art is the mesenchymal stem cell (MSC). MSCs are derived from the embryonal mesoderm and can be isolated from many sources, including adult bone marrow, peripheral blood, fat, placenta, and umbilical blood, among others. MSCs can differentiate into many mesodermal tissues, including muscle, bone, cartilage, fat, and tendon. There is considerable literature on these cells. See, for example, U.S. Patent Nos. 5,486,389; 5,827,735; 5,811,094; 5,736,396; 5,837,539; 5,837,670; and 5,827,740. See also Pittenger, M. et al, Science, 284:143-147 (1999).

Another example of an adult stem cell is adipose-derived adult stem cells (ADSCs) which have been isolated from fat, typically by liposuction followed by release of the ADSCs using collagenase. ADSCs are similar in many ways to MSCs derived from bone marrow, except that it is possible to isolate many more cells from fat. These cells have been reported to differentiate into bone, fat, muscle, cartilage, and neurons. A method of isolation has been described in U.S. 2005/0153442.

Other stem cells that are known in the art include gastrointestinal stem cells, epidermal stem cells, and hepatic stem cells, which have also been termed "oval cells" (Potten, C., et al., Trans R Soc Lond B Biol Sci, 353:821-830 (1998), Watt, F., Trans R Soc Lond B Biol Sci, 353:831 (1997); Alison et al., Hepatology, 29:678-683 (1998).

Other non-embryonic cells reported to be capable of differentiating into cell types of more than one embryonic germ layer include, but are not limited to, cells from umbilical cord blood (see U.S. Publication No. 2002/0164794), placenta (see U.S. Publication No. 2003/0181269, umbilical cord matrix (Mitchell, K.E. et al., Stem Cells, 21:50-60 (2003)), small embryonic-like stem cells (Kucia, M. et al., J Physiol Pharmacol, 57 Suppl 5:5-18 (2006)), amniotic fluid stem cells (Atala, A., J Tissue Regen Med, 1:83-96 (2007)), skin-derived precursors (Toma et al., Nat Cell Biol, 3:778-784 (2001)), and bone marrow (see U.S. Publication Nos. 2003/0059414 and 2006/0147246).

### Strategies of Reprogramming Somatic Cells (for reference)

Several different strategies such as nuclear transplantation, cellular fusion, and culture induced reprogramming have been employed to induce the conversion of differentiated cells into an embryonic state. Nuclear transfer involves the injection of a somatic nucleus into an enucleated oocyte, which, upon transfer into a surrogate mother, can give rise to a clone ("reproductive cloning"), or, upon explantation in culture, can give rise to genetically matched embryonic stem (ES) cells ("somatic cell nuclear transfer," SCNT). Cell fusion of somatic cells with ES cells results in the generation of hybrids that show all features of pluripotent ES cells. Explantation of somatic cells in culture selects for immortal cell lines that may be pluripotent or multipotent. At present, spermatogonial stem cells are the only source of pluripotent cells that can be derived from postnatal animals. Transduction of somatic cells with defined factors can initiate reprogramming to a pluripotent state. These experimental approaches have been extensively reviewed (Hochedlinger and Jaenisch, Nature, 441:1061-1067 (2006) and Yamanaka, S., Cell Stem Cell, 1:39-49 (2007)).

### Nuclear Transfer (for reference)

Nuclear transplantation (NT), also referred to as somatic cell nuclear transfer (SCNT), denotes the introduction of a nucleus from a donor somatic cell into an enucleated ogocyte to generate a cloned animal such as Dolly the sheep (Wilmut et al., Nature, 385:810-813 (1997). The generation of live animals by NT demonstrated that the epigenetic state of somatic cells, including that of terminally differentiated cells, while stable, is not irreversible fixed but can be reprogrammed to an embryonic state that is capable of directing development of a new organism. In addition to providing an exciting experimental approach for elucidating the basic epigenetic mechanisms involved in embryonic development and disease, nuclear cloning technology is of potential interest for patient-specific transplantation medicine.

### Fusion of Somatic Cells and Embryonic Stem Cells (for reference)

Epigenetic reprogramming of somatic nuclei to an undifferentiated state has been demonstrated in murine hybrids produced by fusion of embryonic cells with somatic cells. Hybrids between various somatic cells and embryonic carcinoma cells (Solter, D., Nat Rev Genet, 7:319-327 (2006), embryonic germ (EG), or ES cells (Zwaka and Thomson, Development, 132:227-233 (2005)) share many features with the parental embryonic cells, indicating that the pluripotent phenotype is dominant in such fusion products. As with mouse (Tada et al., Curr Biol, 11:1553-1558 (2001)), human ES cells have the potential to reprogram somatic nuclei after fusion (Cowan et al., Science, 309:1369-1373(2005)); Yu et al., Science, 318:1917-1920 (2006)). Activation of silent pluripotency markers such as Oct4 or reactivation of the inactive somatic X chromosome provided molecular evidence for reprogramming of the somatic genome in the hybrid cells. It has been suggested that DNA replication is essential for the activation of pluripotency markers, which is first observed 2 days after fusion (Do and Scholer, Stem Cells, 22:941-949 (2004)), and that forced overexpression of Nanog in ES cells promotes pluripotency when fused with neural stem cells (Silva et al., Nature, 441:997-1001 (2006)).

### Culture-Induced Reprogramming

Pluripotent cells have been derived from embryonic sources such as blastomeres and the inner cell mass (ICM) of the blastocyst (ES cells), the epiblast (EpiSC cells), primordial germ cells (EG cells), and postnatal spermatogonial stem cells ("maGSCsm" "ES-like" cells). Cells isolated from embryonic stem cells and germ cells are described for reference only. The following pluripotent cells, along with their donor cell/tissue is as follows: parthogenetic ES cells are derived from murine oocytes (Narasimha et al., Curr Biol, 7:881-884 (1997)); embryonic stem cells have been derived from blastomeres (Wakayama et al., Stem Cells, 25:986-993 (2007)); inner cell mass cells (source not applicable) (Eggan et al., Nature, 428:44-49 (2004)); embryonic germ and embryonal carcinoma cells have been derived from primordial germ cells (Matsui et al., Cell, 70:841-847 (1992)); GMCS, maSSC, and MASC have been derived from spermatogonial stem cells (Guan et al., Nature, 440:1199-1203 (2006); Kanatsu-Shinohara et al., Cell, 119:1001-1012 (2004); and Seandel et al., Nature, 449:346-350 (2007)); EpiSC cells are derived from epiblasts (Brons et al., Nature, 448:191-195 (2007); Tesar et al., Nature, 448:196-199(2007)); parthogenetic ES cells have been derived from human oocytes (Cibelli et al., Science, 295L819 (2002); Revazova et al., Cloning Stem Cells, 9:432-449 (2007)); human ES cells have been derived from human blastocysts (Thomson et al., Science, 282:1145-1147 (1998)); MAPC have been derived from bone marrow (Jiang et al., Nature, 418:41-49 (2002); Phinney and Prockop, Stem Cells, 25:2896-2902 (2007)); cord blood cells (derived from cord blood) (van de Ven et al., Exp Hematol, 35:1753-1765 (2007)); neurosphere derived cells derived from neural cell (Clarke et al., Science, 288:1660-1663 (2000)). Donor cells from the germ cell lineage such as PGCs or spermatogonial stem cells are known to be unipotent *in vivo,* but it has been shown that pluripotent ES-like cells (Kanatsu-Shinohara et al., Cell, 119:1001-1012 (2004) or maGSCs (Guan et al., Nature, 440:1199-1203 (2006), can be isolated after prolonged *in vitro* culture. While most of these pluripotent cell types were capable of in vitro differentiation and teratoma formation, only ES, EG, EC, and the spermatogonial stem cell-derived maGCSs or ES-like cells were pluripotent by more stringent criteria, as they were able to form postnatal chimeras and contribute to the germline. Recently, multipotent adult spermatogonial stem cells (MASCs) were derived from testicular spermatogonial stem cells of adult mice, and these cells had an expression profile different from that of ES cells (Seandel et al., Nature, 449:346-350 (2007)) but similar to EpiSC cells, which were derived from the epiblast of postimplantation mouse embryos (Brons et al., Nature, 448:191-195 (2007); Tesar et al., Nature, 448:196-199 (2007)).

### Reprogramming by Defined Transcription Factors

Takahashi and Yamanaka have reported reprogramming somatic cells back to an ES-like state (Takahashi and Yamanaka, Cell, 126:663-676 (2006)). They successfully reprogrammed mouse embryonic fibroblasts (MEFs) and adult fibroblasts to pluripotent ES-like cells after viral-mediated transduction of the four transcription factors Oct4, Sox2, c-myc, and Klf4 followed by selection for activation of the Oct4 target gene Fbx15 (Figure 2A). Cells that had activated Fbx15 were coined iPS (induced pluripotent stem) cells and were shown to be pluripotent by their ability to form teratomas, although they were unable to generate live chimeras. This pluripotent state was dependent on the continuous viral expression of the transduced Oct4 and Sox2 genes, whereas the endogenous Oct4 and Nanog genes were either not expressed or were expressed at a lower level than in ES cells, and their respective promoters were found to be largely methylated. This is consistent with the conclusion that the Fbx15-iPS cells did not correspond to ES cells but may have represented an incomplete state of reprogramming. While genetic experiments had established that Oct4 and Sox2 are essential for pluripotency (Chambers and Smith, Oncogene, 23:7150-7160 (2004); Ivanona et al., Nature, 442:5330538 (2006); Masui et al., Nat Cell Biol, 9:625-635 (2007)), the role of the two oncogenes c-myc and Klf4 in reprogramming is less clear. Some of these oncogenes may, in fact, be dispensable for reprogramming, as both mouse and human iPS cells have been obtained in the absence of c-myc transduction, although with low efficiency (Nakagawa et al., Nat Biotechnol, 26:191-106 (2008); Werning et al., Nature, 448:318-324 (2008); Yu et al., Science, 318: 1917-1920 (2007)).

### MAPC

Human MAPCs are described in U.S. Patent 7,015,037. MAPCs have been identified in other mammals. Murine MAPCs, for example, are also described in U.S. Patent 7,015,037. Rat MAPCs are also described in U.S. Patent No. 7,838,289.

These references describe MAPCs first isolated by Catherine Verfaillie.

### Isolation and Growth of MAPCs

Methods of MAPC isolation are known in the art. See, for example, U.S. Patent 7,015,037, and these methods, along with the characterization (phenotype) of MAPCs. MAPCs can be isolated from multiple sources, including, but not limited to, bone marrow, placenta, umbilical cord and cord blood, muscle, brain, liver, spinal cord, blood or skin. It is, therefore, possible to obtain bone marrow aspirates, brain or liver biopsies, and other organs, and isolate the cells using positive or negative selection techniques available to those of skill in the art, relying upon the genes that are expressed (or not expressed) in these cells (e.g., by functional or morphological assays such as those disclosed in the above-referenced applications).

MAPCs have also been obtained my modified methods described in Breyer et al., Experimental Hematology, 34:1596-1601 (2006) and Subramanian et al., Cellular Programming and Reprogramming: Methods and Protocols; S. Ding (ed.), Methods in Molecular Biology, 636:55-78 (2010).

### MAPCs from Human Bone Marrow as Described in U.S. Patent 7,015,037

MAPCs do not express the common leukocyte antigen CD45 or erythroblast specific glycophorin-A (Gly-A). The mixed population of cells was subjected to a Ficoll Hypaque separation. The cells were then subjected to negative selection using anti-CD45 and anti-Gly-A antibodies, depleting the population of CD45⁺ and Gly-A⁺ cells, and the remaining approximately 0.1% of marrow mononuclear cells were then recovered. Cells could also be plated in fibronectin-coated wells and cultured as described below for 2-4 weeks to deplete the cells of CD45⁺ and Gly-A⁺ cells. In cultures of adherent bone marrow cells, many adherent stromal cells undergo replicative senescence around cell doubling 30 and a more homogenous population of cells continues to expand and maintains long telomeres.

Alternatively, positive selection could be used to isolate cells via a combination of cell-specific markers. Both positive and negative selection techniques are available to those of skill in the art, and numerous monoclonal and polyclonal antibodies suitable for negative selection purposes are also available in the art (see, for example, Leukocyte Typing V, Schlossman, et al., Eds. (1995) Oxford University Press) and are commercially available from a number of sources.

Techniques for mammalian cell separation from a mixture of cell populations have also been described by Schwartz, et al., in U. S. Patent No. 5,759,793 (magnetic separation), Basch et al., 1983 (immunoaffinity chromatography), and Wysocki and Sato, 1978 (fluorescence-activated cell sorting).

Cells may be cultured in low-serum or serum-free culture medium. Serum-free medium used to culture MAPCs is described in U.S. Patent 7,015,037. Commonly-used growth factors include but are not limited to platelet-derived growth factor and epidermal growth factor. See, for example, U.S. Patent Nos. 7,169,610; 7,109,032; 7,037,721; 6,617,161; 6,617,159; 6,372,210;6,224,860; 6,037,174; 5,908,782; 5,766,951; 5,397,706; and 4,657,866; all teach growing cells in serum-free medium.

### Additional Culture Methods

In additional experiments the density at which MAPCs are cultured can vary from about 100 cells/cm² or about 150 cells/cm² to about 10,000 cells/cm², including about 200 cells/cm² to about 1500 cells/cm² to about 2000 cells/cm². The density can vary between species. Additionally, optimal density can vary depending on culture conditions and source of cells. It is within the skill of the ordinary artisan to determine the optimal density for a given set of culture conditions and cells.

Also, effective atmospheric oxygen concentrations of less than about 10%, including about 1-5% and, especially, 3-5%, can be used at any time during the isolation, growth and differentiation of MAPCs in culture.

Cells may be cultured under various serum concentrations, e.g., about 2-20%. Fetal bovine serum may be used. Higher serum may be used in combination with lower oxygen tensions, for example, about 15-20%. Cells need not be selected prior to adherence to culture dishes. For example, after a Ficoll gradient, cells can be directly plated, e.g., 250,000-500,000/cm². Adherent colonies can be picked, possibly pooled, and expanded.

In one instance, used in the experimental procedures in the Examples, high serum (around 15-20%) and low oxygen (around 3-5%) conditions were used for the cell culture. Specifically, adherent cells from colonies were plated and passaged at densities of about 1700-2300 cells/cm² in 18% serum and 3% oxygen (with PDGF and EGF).

In an instance specific for MAPCs, supplements are cellular factors or components that allow MAPCs to retain the ability to differentiate into cell types of more than one embryonic lineage, such as all three lineages. This may be indicated by the expression of specific markers of the undifferentiated state, such as Oct 3/4 (Oct 3A) and/or markers of high expansion capacity, such as telomerase.

### Cell Culture

For all the components listed below, see U.S. 7,015,037.

In general, cells useful for the invention can be maintained and expanded in culture medium that is available and well-known in the art. Also contemplated is supplementation of cell culture medium with mammalian sera. Additional supplements can also be used advantageously to supply the cells with the necessary trace elements for optimal growth and expansion. Hormones can also be advantageously used in cell culture. Lipids and lipid carriers can also be used to supplement cell culture media, depending on the type of cell and the fate of the differentiated cell. Also contemplated is the use of feeder cell layers.

Cells in culture can be maintained either in suspension or attached to a solid support, such as extracellular matrix components. Stem cells often require additional factors that encourage their attachment to a solid support, such as type I and type II collagen, chondroitin sulfate, fibronectin, "superfibronectin" and fibronectin-like polymers, gelatin, poly-D and poly-L-lysine, thrombospondin and vitronectin. The present disclosure may utilize fibronectin. See, for example, Ohashi et al., Nature Medicine, 13:880-885 (2007); Matsumoto et al., J Bioscience and Bioengineering, 105:350-354 (2008); Kirouac et al., Cell Stem Cell, 3:369-381 (2008); Chua et al., Biomaterials, 26:2537-2547 (2005); Drobinskaya et al., Stem Cells, 26:2245-2256 (2008); Dvir-Ginzberg et al., FASEB J, 22:1440-1449 (2008); Turner et al., J Biomed Mater Res Part B: Appl Biomater, 82B:156-168 (2007); and Miyazawa et al., Journal of Gastroenterology and Hepatology, 22:1959-1964 (2007)).

Cells may also be grown in "3D" (aggregated) cultures. An example is PCT/US2009/31528, filed January 21, 2009.

Once established in culture, cells can be used fresh or frozen and stored as frozen stocks, using, for example, DMEM with 40% FCS and 10% DMSO. Other methods for preparing frozen stocks for cultured cells are also available to those of skill in the art.

### Pharmaceutical Formulations

U.S. 7,015,037 teaches pharmaceutical formulations. The cell populations may be present within a composition adapted for and suitable for delivery, i.e., physiologically compatible.

The purity of the cells (or conditioned medium) for administration to a subject may be about 100% (substantially homogeneous). It may be 95% to 100%. It may be 85% to 95%. Particularly, in the case of admixtures with other cells, the percentage can be about 10%-15%, 15%-20%, 20%-25%, 25%-30%, 30%-35%, 35%-40%, 40%-45%, 45%-50%, 60%-70%, 70%-80%, 80%-90%, or 90%-95%. Or isolation/purity can be expressed in terms of cell doublings where the cells have undergone, for example, 10-20, 20-30, 30-40, 40-50 or more cell doublings.

The choice of formulation for administering the cells for a given application will depend on a variety of factors. Prominent among these will be the species of subject, the nature of the condition being treated, its state and distribution in the subject, the nature of other therapies and agents that are being administered, the optimum route for administration, survivability via the route, the dosing regimen, and other factors that will be apparent to those skilled in the art. For instance, the choice of suitable carriers and other additives will depend on the exact route of administration and the nature of the particular dosage form.

Final formulations of the aqueous suspension of cells/medium will typically involve adjusting the ionic strength of the suspension to isotonicity (i.e., about 0.1 to 0.2) and to physiological pH (i.e., about pH 6.8 to 7.5). The final formulation will also typically contain a fluid lubricant.

The cells/medium may be formulated in a unit dosage injectable form, such as a solution, suspension, or emulsion. Pharmaceutical formulations suitable for injection of cells/medium typically are sterile aqueous solutions and dispersions. Carriers for injectable formulations can be a solvent or dispersing medium containing, for example, water, saline, phosphate buffered saline, polyol (for example, glycerol, propylene glycol, liquid polyethylene glycol, and the like), and suitable mixtures thereof.

The skilled artisan can readily determine the amount of cells and optional additives, vehicles, and/or carrier in compositions to be administered in methods described herein. Typically, any additives (in addition to the cells) are present in an amount of 0.001 to 50 wt % in solution, such as in phosphate buffered saline. The active ingredient is present in the order of micrograms to milligrams, such as about 0.0001 to about 5 wt %, preferably about 0.0001 to about 1 wt %, most preferably about 0.0001 to about 0.05 wt % or about 0.001 to about 20 wt %, preferably about 0.01 to about 10 wt %, and most preferably about 0.05 to about 5 wt %.

The cells may be encapsulated for administration, particularly where encapsulation enhances the effectiveness of the therapy, or provides advantages in handling and/or shelf life. Cells may be encapsulated by membranes, as well as capsules, prior to implantation. It is contemplated that any of the many methods of cell encapsulation available may be employed.

A wide variety of materials may be used for microencapsulation of cells. Such materials include, for example, polymer capsules, alginate-poly-L-lysine-alginate microcapsules, barium poly-L-lysine alginate capsules, barium alginate capsules, polyacrylonitrile/polyvinylchloride (PAN/PVC) hollow fibers, and polyethersulfone (PES) hollow fibers.

Techniques for microencapsulation of cells that may be used for administration of cells are known to those of skill in the art and are described, for example, in Chang, P., et al., 1999; Matthew, H.W., et al., 1991; Yanagi, K., et al., 1989; Cai Z.H., et al., 1988; Chang, T.M., 1992 and in U.S. Patent No. 5,639,275 (which, for example, describes a biocompatible capsule for long-term maintenance of cells that stably express biologically active molecules. Additional methods of encapsulation are in European Patent Publication No. 301,777 and U.S. Pat. Nos. 4,353,888; 4,744,933; 4,749,620; 4,814,274; 5,084,350; 5,089,272; 5,578,442; 5,639,275; and 5,676,943.

The cells may be incorporated into a polymer, such as a biopolymer or synthetic polymer. Examples of biopolymers include, but are not limited to, fibronectin, fibrin, fibrinogen, thrombin, collagen, and proteoglycans. Other factors, such as the cytokines discussed above, can also be incorporated into the polymer. Cells may be incorporated in the interstices of a three-dimensional gel. A large polymer or gel, typically, will be surgically implanted. A polymer or gel that can be formulated in small enough particles or fibers can be administered by other common, more convenient, non-surgical routes.

The dosage of the cells will vary within wide limits and will be fitted to the individual requirements in each particular case. In general, in the case of parenteral administration, it is customary to administer from about 0.01 to about 20 million cells/kg of recipient body weight. The number of cells will vary depending on the weight and condition of the recipient, the number or frequency of administrations, and other variables known to those of skill in the art. The cells can be administered by a route that is suitable for the tissue or organ. For example, they can be administered systemically, i.e., parenterally, by intravenous administration, or can be targeted to a particular tissue or organ; they can be administrated via subcutaneous administration or by administration into specific desired tissues.

The cells can be suspended in an appropriate excipient in a concentration from about 0.01 to about 5x10⁶ cells/ml. Suitable excipients for injection solutions are those that are biologically and physiologically compatible with the cells and with the recipient, such as buffered saline solution or other suitable excipients. The composition for administration can be formulated, produced, and stored according to standard methods complying with proper sterility and stability.

### Administration into Lymphohematopoietic Tissues

Techniques for administration into these tissues are known in the art. For example, intra-bone marrow injections can involve injecting cells directly into the bone marrow cavity typically of the posterior iliac crest but may include other sites in the iliac crest, femur, tibia, humerus, or ulna; splenic injections could involve radiographic guided injections into the spleen or surgical exposure of the spleen via laparoscopic or laparotomy; Peyer's patches, GALT, or BALT injections could require laparotomy or laparoscopic injection procedures.

### Dosing

Doses for humans or other mammals can be determined without undue experimentation by the skilled artisan, from this disclosure, the documents cited herein, and the knowledge in the art. The dose of cells/medium appropriate to be used will depend on numerous factors. The parameters that will determine optimal doses to be administered for primary and adjunctive therapy generally will include some or all of the following: the disease being treated and its stage; the species of the subject, their health, gender, age, weight, and metabolic rate; the subject's immunocompetence; other therapies being administered; and expected potential complications from the subject's history or genotype. The parameters may also include: whether the cells are syngeneic, autologous, allogeneic, or xenogeneic; their potency (specific activity); the site and/or distribution that must be targeted for the cells/medium to be effective; and such characteristics of the site such as accessibility to cells/medium and/or engraftment of cells. Additional parameters include co-administration with other factors (such as growth factors and cytokines). The optimal dose in a given situation also will take into consideration the way in which the cells/medium are formulated, the way they are administered, and the degree to which the cells/medium will be localized at the target sites following administration.

The optimal dose of cells could be in the range of doses used for autologous, mononuclear bone marrow transplantation. For fairly pure preparations of cells, optimal doses will range from 10⁴ to 10⁸ cells/kg of recipient mass per administration. The optimal dose per administration may be between 10⁵ to 10⁷ cells/kg. The optimal dose per administration may be 5 x 10⁵ to 5 x 10⁶ cells/kg. By way of reference, higher doses in the foregoing are analogous to the doses of nucleated cells used in autologous mononuclear bone marrow transplantation. Some of the lower doses are analogous to the number of CD34⁺ cells/kg used in autologous mononuclear bone marrow transplantation.

The cells/medium may be administered in an initial dose, and thereafter maintained by further administration. Cells/medium may be administered by one method initially, and thereafter administered by the same method or one or more different methods. The levels can be maintained by the ongoing administration of the cells/medium. The cells/medium may be administered either initially or to maintain their level in the subject or both by intravenous injection. Other forms of administration, may be used, dependent upon the patient's condition and other factors, discussed elsewhere herein.

Cells/medium may be administered in many frequencies over a wide range of times. Generally lengths of treatment will be proportional to the length of the disease process, the effectiveness of the therapies being applied, and the condition and response of the subject being treated.

### Uses

Administering the cells is useful to provide angiogenesis in any number of pathologies, including, but not limited to, any ischemic condition, for example, acute myocardial infarction, chronic heart failure, peripheral vascular disease, stroke, chronic total occlusion, renal ischemia, and acute kidney injury.

Inducers of one or more pro-angiogenic factors can be admixed with the cells to be administered prior to administration or could be co-administered (simultaneous or sequential) with the cells.

In addition, other uses are provided by knowledge of the biological mechanisms described in this application. One of these includes drug discovery. This involves screening one or more compounds for the ability to modulate the expression and/or secretion of one or more pro-angiogenic factors and/or the angiogenic effects of the one or more pro-angiogenic factors secreted by the cells. This would involve an assay for the cell's ability express and/or secrete one or more pro-angiogenic factors and/or the angiogenic effects of the one or more pro-angiogenic factors. Accordingly, the assay may be designed to be conducted *in vivo* or *in vitro.*

Cells (or medium) can be selected by directly assaying factor protein or RNA. This can be done through any of the well-known techniques available in the art, such as by FACS and other antibody-based detection methods and PCR and other hybridization-based detection methods. Indirect assays may also be used for factor expression, such as binding to any of the known receptors. Indirect effects also include assays for any of the specific biological signaling steps/events triggered by binding of a factor to any of its receptors. Therefore, a cell-based assay can also be used. Downstream targets can also be used to assay for expression/secretion of the one or more pro-angiogenic factors. Detection may be direct, e.g., via RNA or protein assays or indirect, e.g., biological assays for one or more biological effects of these factors.

Accordingly, a surrogate marker could be used as long as it serves as an indicator that the cells express/secrete the one or more pro-angiogenic factors.

Assays for expression/secretion include, but are not limited to, ELISA, Luminex. qRT-PCR, anti-factor western blots, and factor immunohistochemistry on tissue samples or cells.

Quantitative determination of the factor(s) in cells and conditioned media can be performed using commercially available assay kits (e.g., R&D Systems that relies on a two-step subtractive antibody-based assay).

*In vitro* angiogenesis assays can also be used to assess the expression/secretion of the factors. Such *in vitro* angiogenesis assays are well known in the art. See, for example, HUVEC tube formation assay as described in this application, endothelial cells proliferation or migration assays, aortic ring assays, and chick chorioallantoic membrane assay (CAM). Assays for angiogenesis *in vivo* may also be applied using any of the well-known assays for determining *in vivo* angiogenesis, such as matrigel plug assays, chick aortic arch assay, and matrigel sponge assays.

A further use is the establishment of cell banks to provide cells for clinical administration. Generally, a fundamental part of this procedure is to provide cells that have a desired potency for administration in various therapeutic clinical settings.

Any of the same assays useful for drug discovery could also be applied to selecting cells for the bank as well as from the bank for administration.

Accordingly, in a banking procedure, the cells (or medium) would be assayed for the ability to achieve any of the above effects. Then, cells would be selected that have a desired potency for any of the above effects, and these cells would form the basis for creating a cell bank.

It is also contemplated that potency can be increased by treatment with an exogenous compound, such as a compound discovered through screening the cells with large combinatorial libraries. These compound libraries may be libraries of agents that include, but are not limited to, small organic molecules, antisense nucleic acids, siRNA DNA aptamers, peptides, antibodies, non-antibody proteins, cytokines, chemokines, and chemo-attractants. For example, cells may be exposed such agents at any time during the growth and manufacturing procedure. The only requirement is that there be sufficient numbers for the desired assay to be conducted to assess whether or not the agent increases potency. Such an agent, found during the general drug discovery process described above, could more advantageously be applied during the last passage prior to banking.

One use that has been applied successfully to MultiStem is as follows. Cells can be isolated from a qualified marrow donor that has undergone specific testing requirements to determine that a cell product that is obtained from this donor would be safe to be used in a clinical setting. The mononuclear cells are isolated using either a manual or automated procedure. These mononuclear cells are placed in culture allowing the cells to adhere to the treated surface of a cell culture vessel. The MAPC cells are allowed to expand on the treated surface with media changes occurring on day 2 and day 4. On day 6, the cells are removed from the treated substrate by either mechanical or enzymatic means and replated onto another treated surface of a cell culture vessel. On days 8 and 10, the cells are removed from the treated surface as before and replated. On day 13, the cells are removed from the treated surface, washed and combined with a cryoprotectant material and frozen, ultimately, in liquid nitrogen. After the cells have been frozen for at least one week, an aliquot of the cells is removed and tested for potency, identity, sterility and other tests to determine the usefulness of the cell bank. These cells in this bank can then be used by thawing them, placing them in culture or use them out of the freeze to treat potential indications.

Another use is a diagnostic assay for efficacy and beneficial clinical effect following administration of the cells. Depending on the indication, there may be biomarkers available to assess. The dosage of cells can be adjusted during treatment according to the effect.

A further use is to assess the efficacy of the cell to achieve any of the above results as a pre-treatment diagnostic that precedes administering the cells to a subject. Moreover, dosage can depend upon the potency of the cells that are being administered. Accordingly, a pre-treatment diagnostic assay for potency can be useful to determine the dose of the cells initially administered to the patient and, possibly, further administered during treatment based on the real-time assessment of clinical effect.

It is also to be understood that the cells can be used to provide angiogenesis not only for purposes of treatment, but also research purposes, both *in vivo* and *in vitro* to understand the mechanism involved in angiogenesis, normally and in diseased models. Angiogenesis assays, *in vivo* or *in vitro,* can be done in the presence of agents known to be involved in angiogenesis. The effect of those agents can then be assessed. These types of assays could also be used to screen for agents that have an effect on the angiogenesis that is promoted by the cells. Accordingly, one could screen for agents in the disease model that reverse the negative effects and/or promote positive effects. Conversely, one could screen for agents that have negative effects in a model of normal angiogenesis.

### Compositions

Also described herein are cell populations with specific potencies for achieving any of the effects described herein. As described above, these populations are established by selecting for cells that have desired potency. These populations are used to make other compositions, for example, a cell bank comprising populations with specific desired potencies and pharmaceutical compositions containing a cell population with a specific desired potency.

The angiogenic potential of the cells can be increased by a combination of TNF-α, IL-1β, and IFN-γ. Exposure of the cells to this combination of factors increases pro-angiogenic gene expression, such as CXCL5, FGF2, and HGF. IL-8 may also be increased in these conditions.

The secretion of pro-angiogenic molecules can also be increased by treatment of cells with a prostaglandin-F analogue latanoprost. Gene expression of pro-angiogenic factors analyzed by RT-PCR shows an increase in HGF, VEGF, KITLG, and IL-8. The biologic prostaglandin-F also increased VEGF A levels.

### EXAMPLES

### Example 1

### Objective

Delivery of exogenous stem cells after ischemic injury has been shown to provide therapeutic benefit through trophic support to injured tissue by regulating immune and inflammatory cells, limiting apoptosis, stimulating neo-angiogenesis, and recruiting host tissue for repair. Previous results suggest that MultiStem's mechanism of benefit in ischemic injury may be, in part, a result of MultiStem's ability to induce neo-vascularization by promoting angiogenesis. Therefore, this study aimed to test whether MultiStem can induce angiogenesis and identify factors responsible for this activity as well as compare the angiogenic activity of MultiStem to MSC.

### Methods and Results

Using a well-established *in vitro* human umbilical vein endothelial cell (HUVEC) angiogenesis assay, the inventors found that conditioned media collected from MultiStem after four days induces angiogenesis *in vitro.* The inventors identified multiple pro-angiogenic factors secreted by MultiStem including VEGF, CXCL5, and IL-8 and found all three factors are necessary for MultiStem induced angiogenesis. Interestingly, CXCL5 and IL-8 were not found to be expressed by cultured bone marrow derived mesenchymal stromal cells (MSC). In contrast to MultiStem, conditioned media alone from MSC was unable to induce angiogenesis in this *in vitro* system.

### Conclusion

MultiStem can induce angiogenesis, in part, through the expression of IL-8, VEGF and CXCL5. This secretion profile is divergent from MSC and these differences are reflected in their functional activities.

### Condensed abstract

Using a well-established angiogenesis assay, the inventors found that conditioned media collected from MultiStem induces angiogenesis *in vitro.* The inventors identified multiple pro-angiogenic factors secreted by MultiStem including VEGF, CXCL5 and IL-8 and found all three factors are necessary for MultiStem induced angiogenesis. Interestingly, CXCL5 and IL-8 were not expressed by cultured bone marrow derived mesenchymal stromal cells (MSC). In contrast to MultiStem, conditioned media from MSC was unable to induce angiogenesis in this *in vitro* system.

Ischemic injury, characterized by the loss of blood flow to tissues or organs, can have devastating consequences as a result of tissue damage and cell death induced by loss of nutrients and oxygen to the ischemic area¹. Acute myocardial infarction (AMI), peripheral vascular disease (PVD) and stroke are three common examples of ischemic injuries that result from loss of blood flow to the heart, limbs, and brain, respectively. These conditions can result in severe long term organ damage, limb amputation and even death from oxygen and nutrient deprivation. Treatment of these conditions often focuses on quick return of blood flow to the injured area to prevent further tissue damage, cell death and to reduce inflammation².

MultiStem®, a large scale expanded adherent multipotent progenitor cell population derived from bone marrow, has been shown to be beneficial in animal models when delivered following ischemic injury such as AMI and PVD³⁻⁶. For example, compared to vehicle controls, delivery of MultiStem into peri-infarct sites following induction of myocardial infarction by direct left anterior descending arterial ligation resulted in improved left ventricular contractile performance, reduced scar area, increased vascular density and improved myocardial energetic characteristics⁷. Mouse and human MultiStem have also been shown to improve limb movement, increase blood flow and capillary density and decreased necrosis in models of critical limb ischemia⁵. Due to low levels of engraftment of MultiStem and minimal differentiation of MultiStem into myocardium or endothelial cells, the benefits of MultiStem for AMI and PVD are believed to be derived from paracrine effects.

Increased vessel density observed in MultiStem treated animals of AMI and PVD compared to vehicle treated controls suggests that MultiStem may be able to induce neo-vascularization by promoting angiogenesis. This activity may be an important mechanism of benefit in treatment of AMI and PVD. Increased vessel density ultimately results in increased blood flow and, hence, oxygen and nutrient delivery to the site of injury⁸⁻⁹.

Numerous studies have shown that stem cells can promote or enhance angiogenesis and neo-vascularization by secreting pro-angiogenic factors such as VEGF¹⁰⁻¹¹. Based on these studies, the inventors hypothesized that MultiStem would also have the capacity to induce angiogenesis. Therefore, MultiStem was examined to determine whether it secretes factors that could promote angiogenesis. Using angiogenic factor immunoblot arrays, conditioned media from MultiStem and MSC cultures established from common donors was tested, demonstrating consistent angiogenic factor expression patterns between the two culture conditions.

Conditioned serum-free media collected from MultiStem induces angiogenesis *in vitro.* Multiple pro-angiogenic factors secreted by MultiStem were identified including VEGF, CXCL5 and IL-8; and immunodepletion studies demonstrated that all three factors are necessary for MultiStem induced angiogenesis. However, none of these factors alone are sufficient to induce angiogenesis.

CXCL5 and IL-8 were not expressed by cultured bone marrow derived mesenchymal stromal cells (MSC). In contrast to MultiStem, conditioned media from MSC was unable to induce angiogenesis in this *in vitro* system. Previous studies have demonstrated that MSC can stabilize vessel formation in vitro and increase vessel density in ischemic animal models, although recent studies have suggested that MSC inhibit angiogenesis and cause endothelial cell death under certain conditions¹¹⁻¹³. The results suggest that MSC do not secrete soluble factors sufficient to maintain angiogenesis in the absence of coculture with endothelial cells. Taken together, these results suggest that MultiStem and MSC have divergent secretion profiles and these differences are reflected in their paracrine activities under various conditions and settings.

### Materials and Methods

### Cell Culture

Human MultiStem was maintained in culture as described previously⁷. MSCs were purchased from Lonza (Walkersville, MD) and expanded in culture as described by the supplier's protocol. For the same donor MultiStem and MSC preparations, adherent cells were isolated and cultured from a fresh bone marrow using the conditions previously described for each cell line¹⁴. Human umbilical vein endothelial cells (HUVECs) (Lonza) were expanded in culture following manufacturers' instructions at a concentration of 2500 cells/cm². HUVECs were used between passage three and five, seeding at 3000 cells/cm2 and cultured for three days, at which time, the confluence is approximately 70-80% prior to use in angiogenesis assay.

### Preparation of serum-free conditioned media (CM)

MultiStem was plated into a tissue culture flask containing MultiStem culture media. Twenty-four hours later, serum containing media was removed, cells were washed with IX PBS and human MultiStem culture media containing growth factors but lacking serum was added. Cells were cultured for 4 days without any media change and on day 4, the serum-free conditioned media was collected, spun down at 1900rpm for 5 min at 4°C, aliquoted, and stored at -80°C.

### Panomics array

Panomics (Fremont, CA) Human Angiogenesis Antibody Array was performed according to the manufacturer's instructions using 2 ml of a 2-fold diluted 4 day serum-free MultiStem conditioned media sample.

### Angiogenesis array

Angiogenesis antibody array (R& D systems, Minneapolis, MN) was performed according to the manufacturer's instructions using 2 ml of a 2-fold diluted 3 day conditioned media samples from MultiStem and MSC derived from the same donor.

### ELISAs

Protein levels of IL-8, VEGF and CXCL5 were determined by ELISA (R&D Systems). All isoforms of VEGF are detected. Error bars are expressed +/- standard deviation.

### VEGF and CXCL8/IL-8 Immunodepletion

Protein A-agarose beads (Santa Cruz, Santa Cruz, CA) were used at a concentration of 75 1 of 50% slurry per 1ml of CM. Mouse anti-human VEGF monoclonal antibody (Santa Cruz) was used at a concentration of 4ug/ml of CM and rabbit anti-human IL-8 polyclonal antibody (Millipore, Billerica, MA) was used at 2ug/ml CM. Normal mouse IgG (Santa Cruz) and ChromPure rabbit IgG (Jackson ImmunoResearch, West Grove, PA) were used as isotype controls.

Protein A-agarose beads were pre-incubated with anti-VEGF, anti-IL-8 antibody or isotype controls overnight at 4°C with rotation followed by 4 washes with ice-cold IX PBS, spinning at 2500rpm, 2 minutes, 4°C. CM was immunodepleted for 2 hours at 4°C with rotation in 6ml aliquots followed by filtration through 0.45 M filter to rid of any residual beads. CM treated with mouse IgG-AC was used as isotype control. Recombinant human VEGF121 (eBioscience, San Diego, CA) and/or VEGF165 (R&D Systems) isoforms were added back into the immunodepleted CM at concentrations ranging from 50 to 1000pg/ml.

### CXCL5/ENA-78 Neutralization

CXCL5 was neutralized using human CXCL5/ENA-78 antibody (R&D Systems) at a concentration of 10µg/ml CM for 2 hours at 4°C with rotation. Normal total goat IgG (Jackson ImmunoResearch,) at a concentration of 10µgg/ml CM was used as an isotype control. Additional controls used are endothelial growth medium (EGM, Lonza) spiked with either human ENA-78 neutralizing antibody or normal total goat IgG at concentration of 10µg/ml.

### Angiogenesis Assay

Growth factor-reduced Matrigel (BD Bioscience, San Jose, CA) was thawed on ice at 4°C overnight and used at a concentration of 6.0-6.5mg/ml, diluted on ice using ice-cold IX PBS. Four-hundred microliters of Matrigel was distributed into the inner wells of a 24-well tissue culture plate and allowed to solidify for 1 hour at 37°C. Addition of Matrigel was done on ice and outer wells of plate were filled with 1ml of IX PBS.

HUVECs were harvested according to the following protocol: Cells were washed with IX PBS followed by a brief rinse with 0.25X trypsin-EDTA and then quenched using the IX PBS (with residual serum) wash. Cells were resuspended in endothelial cell basal media (EBM) and counted. HUVECs were added to the CM, other experimental conditions and controls at a concentration of 55,000 cells/ml/well. Each sample and control was assayed in triplicate. Plates were incubated for 6 hours or 18 hours at 5% CO2 and 37°C to allow for tube formation. Four fields per well were analyzed for a total of 12 fields. Pictures were taken at using 10 X objective. Angiogenesis was scored by counting the number of tubes formed between cells. Results are expressed as average tubes formed per field +/- SEM.

### Results

### MultiStem secretes factors that promotes angiogenesis in vitro

Previous studies have shown that treatment of ischemic injury with MultiStem results in increased vessel density bordering the area of injury compared with vehicle treated controls, suggesting that MultiStem induces neo-vascularization and angiogenesis. To test whether MultiStem secretes factors which promote angiogenesis, an *in vitro* angiogenesis assay using conditioned media from MultiStem was utilized. MultiStem were plated under normal conditions for 24 hours. The cells were then transferred to serum-free conditions to generate conditioned media for four days. This media was then tested for angiogenic activity in an *in vitro* tube formation assay. HUVECs were plated on reduced growth factor Matrigel that was further diluted to a concentration that did not produce any spontaneous angiogenesis with serum-free basal MultiStem media or serum-free endothelial cell media. HUVECs were plated in conditioned media, basal media or endothelial growth media for 18 hours. Angiogenesis was measured as the average number of tubes formed per field of view for each condition. In the absence of any additional factors, the presence of serum alone in basal media can induce angiogenesis. Therefore, serum-free media was used for all the experiments. Robust, complex tube formation was observed in serum containing endothelial cell media while serum-free endothelial cell media or serum-free basal MultiStem media showed virtually no induction of tube formation. The inventors found that serum-free conditioned media from four day cultures of MultiStem induced angiogenesis compared to basal media (Figure1 A,B).

To identify factors secreted by MultiStem that promote angiogenesis and neo-vascularization, MultiStem serum free conditioned media was analyzed on an angiogenesis antibody array (Figure 1 C). Many pro-angiogeneic factors, as well as a few angiostatic factors are secreted by MultiStem into the media. Most notably, VEGF was secreted by MultiStem as was interleukin 8 (IL-8), both of which are potent angiogenic molecules¹⁵⁻¹⁷. CXCL5, another potent angiogenic cytokine, is also secreted by MultiStem (Figure 1 D) ¹⁸. VEGF, CXCL5 and IL-8 are expressed at physiologically active levels in four day MultiStem conditioned media (Figure D-F).

VEGF's role as a key factor involved in the induction of angiogenesis prompted us to examine if VEGF was necessary for MultiStem's pro-angiogenic activity¹⁹⁻²⁰. Using a VEGF antibody, VEGF was immunodepleted from MultiStem conditioned media. In order to ensure that VEGF was truly depleted from the media, the levels of VEGF from the immunodepeleted media were determined using a VEGF ELISA. The levels of VEGF were reduced by more than 95% in the immunodepleted media compared to the conditioned media and IgG alone depleted media (Figure 2 A). CXCL5 levels and IL-8 levels were not affected by VEGF immunodepletion (Figure 2 B,C). In the absence of VEGF, the induction of angiogenesis by MultiStem conditioned media was reduced (Figure 2, Supplemental Figure 1). These results demonstrate that VEGF is necessary in MultiStem conditioned media to induce angiogenesis.

To establish the minimal levels of VEGF required in MultiStem conditioned media to maintain angiogenic activity, increasing amounts of VEGF were added back to immunodepleted media. VEGF-A, the most studied form of VEGF, commonly referred to as VEGF, has multiple isoforms including VEGF 121 and VEGF 165. When these two isoforms were added back separately to the immunodepleted MultiStem conditioned media to determine the minimal amount of VEGF needed to induce angiogenesis (Figure 2A,C), the inventors found that although neither isoform alone was sufficient to completely restore the levels of angiogenesis previously observed with MultiStem conditioned media, 250 pg/ml of VEGF 121 was sufficient to restore some angiogenesis (Figure 2C). For VEGF 165, 50 pg/ml was sufficient to restore some levels of angiogenesis and the addition of more VEGF165 did not increase the levels of angiogenesis by any appreciable amount (Figure 2D).

### CXCL5 and IL-8 are both necessary for normal levels of MultiStem-induced angiogenesis but are not sufficient to induce angiogenesis

Although VEGF is required for angiogenesis, VEGF alone is not sufficient to induce robust angiogenesis at the concentration present in the conditioned media²²⁻²⁴ . The identification of additional angiogenic factors secreted by MultiStem prompted us to examine whether CXCL5 and IL-8 are necessary for MultiStem's angiogenic activity. To test this hypothesis, IL-8 was immunodepleted from MultiStem conditioned media and found to be reduced by 95% (Figure 3). VEGF and CXCL5 levels, measured by ELISA, remained unaffected in the IL-8 immunodepleted media. In the absence of IL-8, the *in vitro* tube formation of the HUVECs using MultiStem conditioned media was reduced by -60 % (Figure 3, Supplemental Figure II). These results suggest that IL-8 is required for the induction of angiogenesis by MultiStem conditioned media, although MultiStem conditioned media still maintains some level of angiogenic activity even in the absence of Il-8. Similarly, blocking CXCL5 activity by the addition of a CXCL5 blocking antibody into the media resulted in a significant decrease in tube formation in the HUVEC angiogenesis assay (Figure 4 A). CXCL5 levels were reduced in the media with the blocking antibody but VEGF and IL-8 levels remain unchanged (Supplemental Figure III). Interestingly, addition of CXCL5 alone, IL-8 alone or both were not sufficient to induce angiogenesis in basal media, suggesting that these factors are necessary for MultiStem induced angiogenesis but not sufficient to induce angiogenesis (Figure 4 B, C).

### MSC express VEGF but are unable to initiate angiogenesis in the HUVEC in vitro assay

In order to assess whether the levels of angiogenesis induced by MultiStem were similar to those induced by other stem cell lines, serum-free conditioned media from MSC was collected and tested for its ability to induce angiogenesis in culture compared with MultiStem. MSC conditioned media did not induce angiogenesis in this assay, even when repeated with MSC from multiple donors (Figure 6A, Supplemental Figure III). Although other reports have shown MSC can induce angiogenesis in *in vitro* HUVEC assays, theses assays were analyzed 4-6 hours after plating, showed incomplete angiogenesis, or used different conditions²⁵⁻²⁷. When angiogenic tube formation was examined at 6 hours, both MSC and MultiStem conditioned medias induced some tube formation. By 24 hours, however, angiogenesis with MSC conditioned media had collapsed but was maintained with the MultiStem conditioned media (Figure 5).

The expression of VEGF, CXCL5 and IL-8 in MSC conditioned media were examined and VEGF was found to be expressed at higher levels than in MultiStem conditioned media, while CXCL5 and IL-8 were not expressed at detectable levels in MSC conditioned media. To confirm that these results were indicative of the secretion profile of MSC rather than an artifact induced by the serum-free culture, the levels of VEGF, IL-8 and CXCL5 expressed by MSC were examined under their normal culture conditions and compared to the protein levels of these factors found in MultiStem conditioned media under MultiStem's culture conditions. For these cell lines, the inventors derived the MSC and MultiStem from the same donor to eliminate any genetic variation between the two lines. Even when these cell types were derived from the same donor, CXCL5 and IL-8 levels were undetectable in MSC conditioned media but expressed at physiologically active levels in MultiStem media (Figure 5B). In order to further examine the secretion profile of these cells line, the inventors compared the secretion profile of MultiStem conditioned media to the secretion profile of MSC conditioned media on angiogenesis antibody arrays (Figure 6A) from the cells derived from the same donor (Figure 6, Supplemental Data I). The data revealed that there were multiple angiogenic and angiostatic factors secreted by MultiStem that were not secreted by MSC, including angiogenin, HGF, IL-8, Leptin, TIMP-4, and IGFBP-1. In contrast, TIMP-1 (25 fold higher) and IGFBP-2 (16 fold higher), were both expressed at much higher levels in MSC compared to MultiStem. Both VEGF and IGFBP-3 were also expressed at consistently higher levels in MSC than MultiStem although only 3-4 X higher. Taken together, these results indicate that MultiStem and MSC have different secretion profiles even when derived from the same donor and these differences are reflected in the functional differences between the cell lines.

### Discussion

Adult stem cells isolated from various tissues including bone marrow, umbilical cord blood and adipose tissues are currently being developed to treat ischemic injuries such as acute myocardial infarction, stroke and peripheral vascular disease²¹⁻³⁰. These injuries induce cellular and tissue damage from the initial loss of oxygen and nutrients in the affected tissue but also from subsequent inflammation in the region. The quick and sustained recovery of blood flow can reduce damage and inflammation within the ischemic region. The original intent of cell based therapies was the regeneration and repair of lost and damaged tissues following injury through the delivery and subsequent differentiation of exogenous stem cells. However, subsequent studies examining the mechanism of benefit for stem cell therapies have shown that many stem cells work primarily through paracrine effects rather than regeneration since many of the cell types are no longer detectable a few days post-delivery³¹⁻³³. The therapeutic hypotheses are that these cell populations can provide trophic support to the injured tissue by regulating immune and inflammatory cells, limiting apoptosis, stimulating neo-angiogenesis, and recruiting host tissue for repair. Benefit is likely derived from a dynamic cascade of these pathways, and different cell populations may exert influences more strongly on certain pathways. Selection of the most appropriate adherent stem cell population for treatment may reflect both potency of a given population for key pathways, as well as time of delivery to effectively mediate the response. It is therefore important to establish standardized assays for these pathways in order to provide comparative data, and then to correlate these *in vitro* surrogates of activity to injury and recovery.

Multipotent adult progenitor cells are an adherent adult stem cell population derived from bone marrow cultures. Previous *in vivo* studies have shown an increase in vessel density in animals treated with MultiStem following ischemic injury^{4, 7}. In this study, we have shown that MultiStem secretes factors that can induce angiogenesis in an *in vitro* tube formation assay. Further analysis revealed that MultiStem secretes a variety of pro-angiogenic factors including VEGF, IL-8, and CXCL5. Two of these factors, CXCL5 and IL-8, are highly differentially secreted by MultiStem compared with MSC, which secrete very little, if any, CXCL5 and IL-8. VEGF, CXCL5, and IL-8 all are required for MultiStem induced angiogenesis. Removal or inhibition of any of these factors greatly reduces the ability of MultiStem conditioned media to promote angiogenesis. VEGF165 is the major isoform involved in angiogenesis. However, multiple VEGF isoforms may be responsible for MultiStem induced angiogenesis since tube formation could not be restored to 100% using either VEGF121 or VEGF165 isoforms independently in VEGF immunodepleted MultiStem conditioned media.

Although other groups have delivered single pro-angiogenic factors such as VEGF to ischemic injury models to provide some beneficial effect in animals, the results have been mixed for clinical indications such as AMI and PVD^{34, 35}. Uncontrolled expression of angiogenic factors can lead to serious side effects such as hemangioma formation, arthritis and retinopathy, and severe pleural effusion and pericardial effusion in the rat AMI model^{14, 36, 37}. The results of clinical trials for single angiogenic factors by gene or protein delivery have been disappointing for PVD most likely due to multiple factors including instability of currently tested factors which are required for long term benefit, delivery complications, low uptake and response of ischemic tissue and requirement for several concurrent molecules to achieve functional revascularization. In contrast, treatment of ischemic injuries with stem cells could offer an attractive alternative to single protein or gene treatment. The use of stem cells to treat ischemic injuries could result in the delivery of multiple angiogenic factors directly to the site of injury, by cells that respond and home to the hypoxic and inflammatory microenvironment, achieving a dynamic balance to stimulate an appropriate angiogenic response. Additionally, stem cells such as MultiStem could also simultaneously prevent tissue damage through immunomodulatory and anti-apoptotic mechanisms. In this study, the inventors demonstrate that MultiStem is, indeed, capable of inducing angiogenesis directly through the expression of at least three pro-angiogenic factors.

Although MSC express and secrete high levels of VEGF, conditioned media from MSC was insufficient to induce angiogenesis in this *in vitro* assay system. MSC have been shown to stabilize vessel formation *in vitro* in previous studies. However, many of these studies examine vessel formation at an earlier time point, such as at 4-6 hours or under different conditions^{25-27, 38}. The inventors found that at these earlier time points, there is a higher level of background of angiogenesis in negative controls which is not stable at 24 hours. Similarly, we found at 6 hours, MSC can induce some level of angiogenesis which is subsequently lost by the 24 hour time point. In contrast, MultiStem induces tube formation that remains stable at 24 hours. These results suggest that although MSC can support angiogenesis in the short term, in the absence of other factors, this vessel formation is not stable. These results reflect data from earlier studies that VEGF alone is not sufficient to initiate stable vessel formation at the levels expressed by these cells²⁴. In the context of previous *in vivo* experiments which show increased vessel density in ischemic injuries treated with MSC, MSC may increase vessel density by inducing endogenous inflammatory cells or tissue progenitors to promote angiogenesis.

### References

1. Rosamond, W. et al., "Heart disease and stroke statistics--2007 update: a report from the American Heart Association Statistics Committee and Stroke Statistics Subcommittee" Circulation, Feb 6 2007;115(5):e69-171.
2. Molin, D and Post, MJ., "Therapeutic angiogenesis in the heart: protect and serve" Current opinion in pharmacology. Apr 2007;7(2):158-163.
3. Kovacsovics-Bankowski, M. et al. "Clinical scale expanded adult pluripotent stem cells prevent graft-versus-host disease" Cellular immunology. 2009;255(1-2):55-60.
4. Pelacho, B. et al., "Multipotent adult progenitor cell transplantation increases vascularity and improves left ventricular function after myocardial infarction" Journal of tissue engineering and regenerative medicine. Jan-Feb 2007;1(1):51-59.
5. Aranguren, XLM et al., "Multipotent adult progenitor cells sustain function of ischemic limbs by stimulating vessel and muscle regeneration" 2007.
6. Kovacsovics-Bankowski, M. et al., "Pre-clinical safety testing supporting clinical use of allogeneic multipotent adult progenitor cells" Cytotherapy. 2008;10(7):730-742.
7. Van't Hof, W. et al., "Direct delivery of syngeneic and allogeneic large-scale expanded multipotent adult progenitor cells improves cardiac function after myocardial infarct" Cytotherapy. 2007;9(5):477-487.
8. van der Laan, A.M. et al., "Targeting angiogenesis to restore the microcirculation after reperfused MI" Nat Rev Cardiol. Jun 16 2009.
9. Idris, N.M. et al., "Therapeutic angiogenesis for treatment of peripheral vascular disease" Growth factors (Chur, Switzerland). Dec 2004;22(4):269-279.
10. Markel, T.A. et al., "VEGF is critical for stem cell-mediated cardioprotection and a crucial paracrine factor for defining the age threshold in adult and neonatal stem cell function" Am J Physiol Heart Circ Physiol. Dec 2008;295(6):H2308-2314.
11. Payne, T.R. et al., "A relationship between vascular endothelial growth factor, angiogenesis, and cardiac repair after muscle stem cell transplantation into ischemic hearts" J Am Coll Cardiol. Oct 23 2007;50(17):1677-1684.
12. Kinnaird, T., et al. "Local delivery of marrow-derived stromal cells augments collateral perfusion through paracrine mechanisms" Circulation. Mar 30 2004;109(12):1543-1549.
13. Tang, Y.L. et al., "Paracrine action enhances the effects of autologous mesenchymal stem cell transplantation on vascular regeneration in rat model of myocardial infarction" Ann Thorac Surg. Jul 2005;80(1):229-236; discussion 236-227.
14. Boozer, S. et al., "Global Characterization and Genomic Stability of MultiStem, a Multipotent Adult Progenitor Cell" Journal of Stem Cells. 2009;4(1):17-28.
15. Koch, A.E. et al., "Regulation of angiogenesis by the C-X-C chemokines interleukin-8 and epithelial neutrophil activating peptide 78 in the rheumatoid joint" Arthritis and rheumatism. Jan 2001;44(1):31-40.
16. Strieter, R.M. et al., "Interleukin-8. A corneal factor that induces neovascularization" The American journal of pathology. Dec 1992;141(6):1279-1284.
17. Keeley, E.C. et al., "Chemokines as mediators of neovascularization" Arterioscler Thromb Vasc Biol. Nov 2008;28(11):1928-1936.
18. Keane, M.P. et al., "ENA-78 is an important angiogenic factor in idiopathic pulmonary fibrosis" American journal of respiratory and critical care medicine. Dec 15 2001;164(12):2239-2242.
19. Carmeliet, P., "Angiogenesis in health and disease" Nature medicine. Jun 2003;9(6):653-660.
20. Carmeliet, P., "Mechanisms of angiogenesis and arteriogenesis" Nature medicine. Apr 2000;6(4):389-395.
21. Neufeld, G. et al., "Vascular endothelial growth factor (VEGF) and its receptors" Faseb J. Jan 1999;13(1):9-22.
22. Kinnunen, K. et al., "Overexpression of VEGF-A induces neovascularization and increased vascular leakage in rabbit eye after intravitreal adenoviral gene transfer" Acta physiologica (Oxford, England). Aug 2006;187(4):447-457.
23. Milkiewicz, M. et al., "Vascular endothelial growth factor mRNA and protein do not change in parallel during non-inflammatory skeletal muscle ischaemia in rat" The Journal of physiology. Dec 1 2006;577(Pt 2):671-678.
24. Loffredo, F. and Lee, R.T., "Therapeutic vasculogenesis: it takes two" Circulation research. Jul 18 2008;103(2):128-130.
25. Iwase, T. et al., "Comparison of angiogenic potency between mesenchymal stem cells and mononuclear cells in a rat model of hindlimb ischemia" Cardiovasc Res. Jun 1 2005;66(3):543-551.
26. Zacharek, A. et al., "Angiopoietinl/Tie2 and VEGF/Flkl induced by MSC treatment amplifies angiogenesis and vascular stabilization after stroke" J Cereb Blood Flow Metab. Oct 2007;27(10):1684-1691.
27. Hung, S.C. et al., "Angiogenic effects of human multipotent stromal cell conditioned medium activate the PI3K-Akt pathway in hypoxic endothelial cells to inhibit apoptosis, increase survival, and stimulate angiogenesis" Stem cells (Dayton, Ohio). Sep 2007;25(9):2363-2370.
28. Burns, T.C. and Verfaillie, C.M., "Low WC. Stem cells for ischemic brain injury: a critical review" The Journal of comparative neurology. Jul 1 2009;515(1):125-144.
29. Singh, S. et al., "Stem cells improve left ventricular function in acute myocardial infarction" Clinical cardiology. Apr 2009;32(4):176-180.
30. Aranguren, X.L. et al., "Emerging hurdles in stem cell therapy for peripheral vascular disease" Journal of molecular medicine (Berlin, Germany). Jan 2009;87(1):3-16.
31. Zhang, M. et al., "SDF-1 expression by mesenchymal stem cells results in trophic support of cardiac myocytes after myocardial infarction" Faseb J. Oct 2007; 21(12):3197-3207.
32. Block, G.J. et al., "Multipotent Stromal Cells (MSCs) are Activated to Reduce Apoptosis in Part by Upregulation and Secretion of Stanniocalcin-1 (STC-1)" Stem cells (Dayton, Ohio). Dec 18 2008.
33. Gnecchi, M. et al., "Paracrine mechanisms in adult stem cell signaling and therapy" Circulation research. Nov 21 2008;103(11):1204-1219.
34. Makinen, K. et al., "Increased vascularity detected by digital subtraction angiography after VEGF gene transfer to human lower limb artery: a randomized, placebo-controlled, double-blinded phase II study" Mol Ther. Jul 2002;6(1):127-133.
35. Rajagopalan, S. et al., "Regional angiogenesis with vascular endothelial growth factor in peripheral arterial disease: a phase II randomized, double-blind, controlled study of adenoviral delivery of vascular endothelial growth factor 121 in patients with disabling intermittent claudication" Circulation. Oct 21 2003;108(16):1933-1938.
36. Su, H. et al., "Adeno-associated viral vector-mediated hypoxia response element-regulated gene expression in mouse ischemic heart model" Proceedings of the National Academy of Sciences of the United States of America. Jul 9 2002;99(14):9480-9485.
37. Schwarz, E.R. et al., "Evaluation of the effects of intramyocardial injection of DNA expressing vascular endothelial growth factor (VEGF) in a myocardial infarction model in the rat--angiogenesis and angioma formation" JAm Coll Cardiol. Apr 2000;35(5):1323-1330.
38. Oswald, J. et al., "Mesenchymal stem cells can be differentiated into endothelial cells in vitro" Stem cells (Dayton, Ohio). 2004;22(3):377-384.

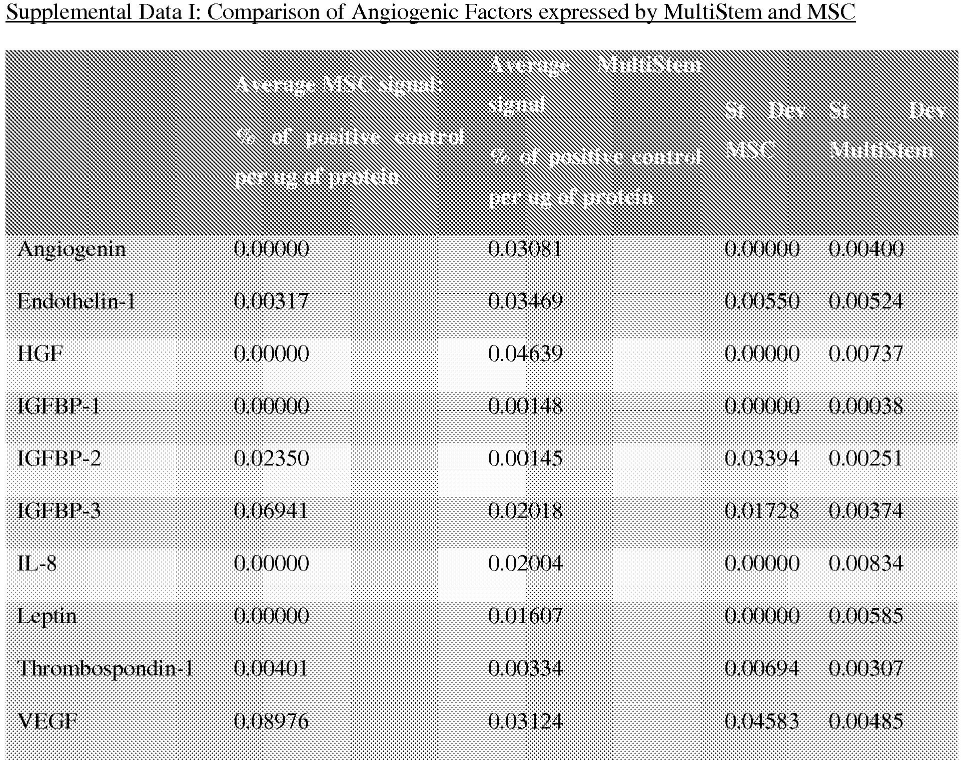

### Example 2

### Enhanced Expression of Angiogenic Factors in MultiStem

Figures 7-12 show that expression of angiogenic factors can be increased in the MultiStem (MAPC) preparation.

## Claims

1. Cells having expression and/or secretion of the pro-angiogenic factors VEGF, CXCL5 and IL-8 for use in preventing or treating an ischemic condition in a subject, the cells being non-embryonic stem, non-germ cells that express one or more of oct4, telomerase, rex-1, or rox-1 and can differentiate into cell types of at least two of endodermal, ectodermal, and mesodermal germ layers, wherein, prior to administration, said cells have been assayed for having expression and/or secretion of the pro-angiogenic factors VEGF, CXCL5 and IL-8.

2. The cells for the use of claim 1, wherein the cells are allogeneic.

3. The cells for the use of any one of claims 1 to 2, wherein the ischemic condition is a condition selected from the group consisting of acute myocardial infarction, chronic heart failure, peripheral vascular disease, stroke, chronic total occlusion, renal ischemia, and acute kidney injury.

4. The cells for the use of any of claims 1-3 wherein said subject is human.

5. A method for constructing a cell bank, said method comprising assaying cells for having expression and/or secretion of the pro-angiogenic factors VEGF, CXCL5 and IL-8, expanding and storing, for future administration to a subject, cells that have the expression and/or secretion of the pro-angiogenic factors VEGF, CXCL5 and IL-8, the cells being non-embryonic stem, non-germ cells that express one or more of oct4, telomerase, rex-1, or rox-1 and can differentiate into cell types of at least two of endodermal, ectodermal, and mesodermal germ layers.

6. An *in vitro* method for drug discovery, said method comprising exposing cells that have expression and/or secretion of the pro-angiogenic factors VEGF, CXCL5 and IL-8, to an agent to assess the effect of the agent on the ability of the cells to express and secrete the pro-angiogenic factors VEGF, CXCL5 and IL-8, the cells being non-embryonic stem, non-germ cells that express one or more of oct4, telomerase, rex-1, or rox-1 and/or can differentiate into cell types of at least two of endodermal, ectodermal, and mesodermal germ layers.

7. The method of claim 6, wherein said cells have been assayed for having expression and/or secretion of the pro-angiogenic factors VEGF, CXCL5 and IL-8.

8. An *in vitro* method for increasing the expression of the pro-angiogenic factors VEGF, CXCL5 and IL-8 in a stem cell, the method comprising exposing the cell to a combination of TNF-α, IL-1β, and IFNγ, or to a prostaglandin F analog wherein said cells are non-embryonic stem, non-germ cells that express one or more of oct4, telomerase, rex-1, or rox-1 and can differentiate into cell types of at least two of endodermal, ectodermal, and mesodermal germ layers, wherein the prostaglandin F analog is latanoprost.

9. The cells for the use of any one of claims 1 to 4 or the method of any one of claims 5 to 8, wherein the cells are multipotent progenitor cells.

10. The cells for the use of any one of claims 1 to 4 and 9, or the method of any one of claims 5 to 9, wherein the cells express telomerase.

11. The cells for the use of any one of claims 1 to 4 and 9, or the method of any one of claims 5 to 9, wherein the cells express oct4.

12. The cells for the use of any one of claims 1 to 4 and 9, or the method of any one of claims 5 to 9, wherein the cells express telomerase and oct4.

13. The cells for the use of any one of claims 1 to 4 and 9 to 12, or the method of any one of claims 5 to 12, wherein the cells can differentiate into cell types of endodermal, ectodermal, and mesodermal germ layers.

14. The cells for the use of any one of claims 1 to 4 and 9 to 13, or the method of any one of claims 5 to 13, wherein the cells are human cells.

15. The cells for the use of any one of claims 1 to 4 and 9 to 14, or the method of any one of claims 5 to 14, wherein the cells are derived from bone marrow.

## Patentansprüche

1. Zellen, die die pro-angiogenen Faktoren VEGF, CXCL5 und IL-8 exprimieren und/oder sezernieren, zur Verwendung bei der Prävention oder Behandlung eines ischämischen Zustands in einem Individuum, wobei die Zellen nicht-embryonale Stamm-, Nicht-Keim-Zellen sind, die ein oder mehr von oct4, Telomerase, rex-1, oder rox-1 exprimieren und zu Zelltypen von mindestens zwei von endodermalen, ektodermalen und mesodermalen Keimschichten differenzieren können, wobei die Zellen vor der Verabreichung dahingehend untersucht wurden, ob sie die pro-angiogenen Faktoren VEGF, CXCL5 und IL-8 exprimieren und/oder sezernieren.

2. Zellen zur Verwendung nach Anspruch 1, wobei die Zellen allogen sind.

3. Zellen zur Verwendung nach einem der Ansprüche 1 bis 2, wobei der ischämische Zustand ein Zustand ist, ausgewählt aus der Gruppe, bestehend aus akutem Myokardinfarkt, chronischem Herzversagen, peripherer Gefäßerkrankung, Schlaganfall, chronischem Totalverschluss, Nierenischämie und akuter Nierenverletzung.

4. Zellen zur Verwendung nach einem der Ansprüche 1 bis 3, wobei das Individuum ein Mensch ist.

5. Verfahren zum Konstruieren einer Zellbank, wobei das Verfahren das Untersuchen der Zellen auf Expression und/oder Sekretion der pro-angiogenen Faktoren VEGF, CXCL5 und IL-8, Vermehren und Aufbewahren, für die zukünftige Verabreichung von Zellen, die die pro-angiogenen Faktoren VEGF, CXCL5 und IL-8 exprimieren und/oder sezernieren, an ein Individuum umfasst, wobei die Zellen nicht-embryonale Stamm-, Nicht-Keim-Zellen sind, die ein oder mehr von oct4, Telomerase, rex-1, oder rox-1 exprimieren und zu Zelltypen von mindestens zwei von endodermalen, ektodermalen und mesodermalen Keimschichten differenzieren können.

6. *In* vitro-Verfahren zur Arzneimittelentdeckung, wobei das Verfahren das Aussetzen von Zellen, die die pro-angiogenen Faktoren VEGF, CXCL5 und IL-8 exprimieren und/oder sezernieren, gegenüber einem Mittel, um die Wirkung des Mittels auf die Fähigkeit von Zellen zu bestimmen, die pro-angiogenen Faktoren VEGF, CXCL5 und IL-8 zu exprimieren und sezernieren, umfasst, wobei die Zellen nicht-embryonale Stamm-, Nicht-Keim-Zellen sind, die ein oder mehr von oct4, Telomerase, rex-1, oder rox-1 exprimieren und/oder zu Zelltypen von mindestens zwei von endodermalen, ektodermalen und mesodermalen Keimschichten differenzieren können.

7. Verfahren nach Anspruch 6, wobei die Zellen dahingehend untersucht wurden, ob sie die pro-angiogenen Faktoren VEGF, CXCL5 und IL-8 exprimieren und/oder sezernieren.

8. In vitro-Verfahren zur Steigerung der Expression der pro-angiogenen Faktoren VEGF, CXCL5 und IL-8 in einer Stammzelle, wobei das Verfahren das Aussetzen der Zelle gegenüber einer Kombination von TNF-α, IL-1β und IPNγ, oder einem Prostaglandin F-Analogon umfasst, wobei die Zellen nicht-embryonale Stamm-, Nicht-Keim-Zellen sind, die ein oder mehr von oct4, Telomerase, rex-1, oder rox-1 exprimieren und zu Zelltypen von mindestens zwei von endodermalen, ektodermalen und mesodermalen Keimschichten differenzieren können, wobei das Prostaglandin F-Analogon Latanoprost ist.

9. Zellen zur Verwendung nach einem der Ansprüche 1 bis 4 oder Verfahren nach einem der Ansprüche 5 bis 8, wobei die Zellen multipotente Vorläuferzellen sind.

10. Zellen zur Verwendung nach einem der Ansprüche 1 bis 4 und 9, oder Verfahren nach einem der Ansprüche 5 bis 9, wobei die Zellen Telomerase exprimieren.

11. Zellen zur Verwendung nach einem der Ansprüche 1 bis 4 und 9, oder Verfahren nach einem der Ansprüche 5 bis 9, wobei die Zellen oct4 exprimieren.

12. Zellen zur Verwendung nach einem der Ansprüche 1 bis 4 und 9, oder Verfahren nach einem der Ansprüche 5 bis 9, wobei die Zellen Telomerase und oct4 exprimieren.

13. Zellen zur Verwendung nach einem der Ansprüche 1 bis 4 und 9 bis 12, oder Verfahren nach einem der Ansprüche 5 bis 12, wobei die Zellen zu Zelltypen endodermaler, ektodermaler und mesodermaler Keimschichten differenzieren können.

14. Zellen zur Verwendung nach einem der Ansprüche 1 bis 4 und 9 bis 13, oder Verfahren nach einem der Ansprüche 5 bis 13, wobei die Zellen menschliche Zellen sind.

15. Zellen zur Verwendung nach einem der Ansprüche 1 bis 4 und 9 bis 14, oder Verfahren nach einem der Ansprüche 5 bis 14, wobei die Zellen aus Knochenmark stammen.

## Revendications

1. Cellules présentant une expression et/ou sécrétion des facteurs pro-angiogéniques VEGF, CXCL5 et IL-8 pour utilisation dans la prévention ou le traitement d'une affection ischémique chez un sujet, les cellules étant des cellules souches non embryonnaires, des cellules non germinales qui expriment l'un ou plusieurs parmi oct4, la télomérase, rex-1 ou rox-1 et peuvent se différencier en types de cellules d'au moins deux parmi des couches germinales endodermiques, ectodermiques et mésodermiques, où, avant administration, lesdites cellules ont été analysées pour l'expression et/ou la sécrétion des facteurs pro-angiogéniques VEGF, CXCL5 et IL-8.

2. Cellules pour l'utilisation de la revendication 1, les cellules étant allogéniques.

3. Cellules pour l'utilisation de l'une quelconque des revendications 1 à 2, l'affection ischémique étant une affection choisie dans le groupe constitué d'un infarctus du myocarde aigu, une insuffisance cardiaque chronique, une maladie vasculaire périphérique, un accident vasculaire cérébral, une occlusion totale chronique, une ischémie rénale et une lésion rénale aiguë.

4. Cellules pour l'utilisation de l'une quelconque des revendications 1 à 3, ledit sujet étant un humain.

5. Procédé de construction d'une banque de cellules, ledit procédé comprenant l'analyse de cellules pour l'expression et/ou la sécrétion des facteurs pro-angiogéniques VEGF, CXCL5 et IL-8, l'expansion et le stockage, pour administration future à un sujet, de cellules qui présentent l'expression et/ou la sécrétion des facteurs pro-angiogéniques VEGF, CXCL5 et IL-8, les cellules étant des cellules souches non embryonnaires, des cellules non germinales qui expriment l'un ou plusieurs parmi oct4, la télomérase, rex-1 ou rox-1 et peuvent se différencier en types de cellules d'au moins deux parmi des couches germinales endodermiques, ectodermiques et mésodermiques.

6. Procédé *in vitro* de découverte de médicament, ledit procédé comprenant l'exposition de cellules qui présentent une expression et/ou sécrétion des facteurs pro-angiogéniques VEGF, CXCL5 et IL-8, à un agent pour évaluer l'effet de l'agent sur la capacité des cellules à exprimer et sécréter les facteurs pro-angiogéniques VEGF, CXCL5 et IL-8, les cellules étant des cellules souches non embryonnaires, des cellules non germinales qui expriment l'un ou plusieurs parmi oct4, la télomérase, rex-1 ou rox-1 et/ou peuvent se différencier en types de cellules d'au moins deux parmi des couches germinales endodermiques, ectodermiques et mésodermiques.

7. Procédé de la revendication 6, dans lequel lesdites cellules ont été analysées pour l'expression et/ou la sécrétion des facteurs pro-angiogéniques VEGF, CXCL5 et IL-8.

8. Procédé *in vitro* pour augmenter l'expression des facteurs pro-angiogéniques VEGF, CXCL5 et IL-8 dans une cellule souche, le procédé comprenant l'exposition de la cellule à une combinaison de TNF-α, IL-1β, et IPNγ, ou à un analogue de prostaglandine F, dans lequel lesdites cellules sont des cellules souches non embryonnaires, des cellules non germinales qui expriment l'un ou plusieurs parmi oct4, la télomérase, rex-1 ou rox-1 et peuvent se différencier en types de cellules d'au moins deux parmi des couches germinales endodermiques, ectodermiques et mésodermiques, dans lequel l'analogue de prostaglandine F est le latanoprost.

9. Cellules pour l'utilisation de l'une quelconque des revendications 1 à 4 ou le procédé de l'une quelconque des revendications 5 à 8, les cellules étant des cellules progénitrices multipotentes.

10. Cellules pour l'utilisation de l'une quelconque des revendications 1 à 4 et 9, ou le procédé de l'une quelconque des revendications 5 à 9, les cellules exprimant la télomérase.

11. Cellules pour l'utilisation de l'une quelconque des revendications 1 à 4 et 9, ou le procédé de l'une quelconque des revendications 5 à 9, les cellules exprimant oct4.

12. Cellules pour l'utilisation de l'une quelconque des revendications 1 à 4 et 9, ou le procédé de l'une quelconque des revendications 5 à 9, les cellules exprimant la télomérase et oct4.

13. Cellules pour l'utilisation de l'une quelconque des revendications 1 à 4 et 9 à 12, ou le procédé de l'une quelconque des revendications 5 à 12, les cellules pouvant se différencier en types de cellules de couches germinales endodermiques, ectodermiques et mésodermiques.

14. Cellules pour l'utilisation de l'une quelconque des revendications 1 à 4 et 9 à 13, ou le procédé de l'une quelconque des revendications 5 à 13, les cellules étant des cellules humaines.

15. Cellules pour l'utilisation de l'une quelconque des revendications 1 à 4 et 9 à 14, ou le procédé de l'une quelconque des revendications 5 à 14, les cellules étant dérivées de moelle osseuse.
